# EUROPEAN PATENT SPECIFICATION

(11) **EP 4 767 926 B1**
(45) Date of publication and mention of the grant of the patent: **25.11.2026**
(21) Application number: 24856412.2
(22) Date of filing: 16.08.2024
(51) Int. Cl.: A61B 5/02, A61B 5/00

(54) **PULSE WAVE MEASUREMENT DEVICE**
PULSWELLENMESSGERÄT
DISPOSITIF DE MESURE D'ONDE PULSATILE

(30) Priority: 24.08.2023 JP 2023136423
(43) Date of publication of application: 01.07.2026
(73) Proprietor: Minebea Mitsumi Inc., Kitasaku-gun, Nagano 3890293 (JP)
(72) Inventor: NAGAI, Takuya, Kitasaku-gun, Nagano 389-0293 (JP); HARADA, Koshi, Kitasaku-gun, Nagano 389-0293 (JP); OKA, Hiroyuki, Kitasaku-gun, Nagano 389-0293 (JP)
(74) Representative: Dennemeyer & Associates S.A.
(86) International application number: PCT/JP2024/029212
(87) International publication number: WO 2025/041723

(56) References cited:
- CN-A- 110 179 447
- CN-A- 110 179 447
- CN-A- 113 729 632
- CN-A- 114 652 410
- CN-U- 217 566 173
- DE-T2- 60 318 254
- JP-A- 2009 240 511
- JP-A- 2021 524 792
- JP-A- 2023 085 754
- JP-A- 2023 085 754
- US-A1- 2021 228 102

## Description

### TECHNICAL FIELD

The present invention relates to a pulse wave measuring device.

### BACKGROUND ART

A pulse wave measuring device including a pulse wave sensor for detecting a pulse wave generated when a heart pumps blood is known. Such a pulse wave measuring device is attachable to, for example, the wrist of a subject (for example, see PTL 1).

### CITATION LIST

### PATENT LITERATURE

PTL 1: Japanese Patent No. 5094131; PTL 2: JP 2023085754 A;

### SUMMARY OF THE INVENTION

### TECHNICAL PROBLEM

Since a pulse wave sensor needs to detect weak signals, it is preferable to place a pulse wave sensor horizontal with respect to the radial artery of a subject in order to improve measurement accuracy.

An object of the present invention is to provide a pulse wave measuring device having a structure in which a pulse wave sensor is hardly inclined with respect to the radial artery of a subject.

### SOLUTION TO THE PROBLEM

A pulse wave measuring device according to an embodiment of the present disclosure includes a sensor part including a pulse wave sensor, and an attaching part connected outside of the sensor part and attachable to a subject, wherein the attaching part includes a first curved member and a second curved member that are curved in opposite directions and face each other to be attachable to a wrist of the subject, and can shift between a closed state and an opened state, wherein the sensor part is disposed at one end of the first curved member in a longitudinal direction of the first curved member, the second curved member includes a region of which a width in a transverse direction of the second curved member is narrower than a width of the first curved member in a transverse direction of the first curved member, and the second curved member has an asymmetric shape with respect to an imaginary line that bisects a maximum width part of the second curved member in the transverse direction and extends in a longitudinal direction of the second curved member.

### ADVANTAGEOUS EFFECTS OF THE INVENTION

According to the disclosed technology, it is possible to provide a pulse wave measuring device having a structure in which a pulse wave sensor is hardly inclined with respect to the radial artery of a subject.

### BRIEF DESCRIPTION OF THE DRAWINGS

[FIG. 1] FIG. 1 is an oblique view (part 1) illustrating a pulse wave measuring device according to a first embodiment.
[FIG. 2] FIG. 2 is an oblique view (part 2) illustrating the pulse wave measuring device according to the first embodiment.
[FIG. 3] FIG. 3 is a side view illustrating the pulse wave measuring device according to the first embodiment.
[FIG. 4] FIG. 4 is a front view illustrating the pulse wave measuring device according to the first embodiment.
[FIG. 5] FIG. 5 is a bottom view illustrating the pulse wave measuring device according to the first embodiment.
[FIG. 6] FIG. 6 is an example of a pulse wave.
[FIG. 7] FIG. 7 is an oblique view illustrating a first curved member.
[FIG. 8] FIG. 8 is an exploded oblique view illustrating a movable part.
[FIG. 9] FIG. 9 is an exploded oblique view illustrating a fixed part and the movable part.
[FIG. 10] FIG. 10 is an exploded oblique view illustrating a sensor part.
[FIG. 11] FIG. 11 is a cross-sectional view illustrating the sensor part.
[FIG. 12] FIG. 12 is a cross-sectional view (part 1) for explaining a pivot part.
[FIG. 13] FIG. 13 is a cross-sectional view (part 2) for explaining the pivot part.
[FIG. 14] FIG. 14 is a plan view illustrating a pulse wave sensor according to the first embodiment.
[FIG. 15] FIG. 15 is a cross-sectional view illustrating the pulse wave sensor according to the first embodiment.
[FIG. 16] FIG. 16 is an example of a bridge circuit.
[FIG. 17] FIG. 17 is a plan view illustrating a strain gauge according to the first embodiment.
[FIG. 18] FIG. 18 is a cross-sectional view (part 1) illustrating a strain gauge according to the first embodiment.
[FIG. 19] FIG. 19 is a cross-sectional view (part 2) illustrating the strain gauge according to the first embodiment.
[FIG. 20] FIG. 20 is a view (part 1) for explaining a biasing member.
[FIG. 21] FIG. 21 is a view (part 2) for explaining the biasing member.
[FIG. 22] FIG. 22 is a cross-sectional view (part 1) illustrating a state in which a cover member is attached to a pulse wave sensor.
[FIG. 23] FIG. 23 is a cross-sectional view (part 2) illustrating a state in which a cover member is attached to a pulse wave sensor.

### DETAILED DESCRIPTION OF THE INVENTION

The invention is as defined in the appended claims. Hereinafter, an embodiment for carrying out the invention will be described with reference to the drawings. In the drawings, the same components are denoted by the same reference numerals, and redundant description may be omitted.

### <First Embodiment>

### [Pulse Wave Measuring Device 1]

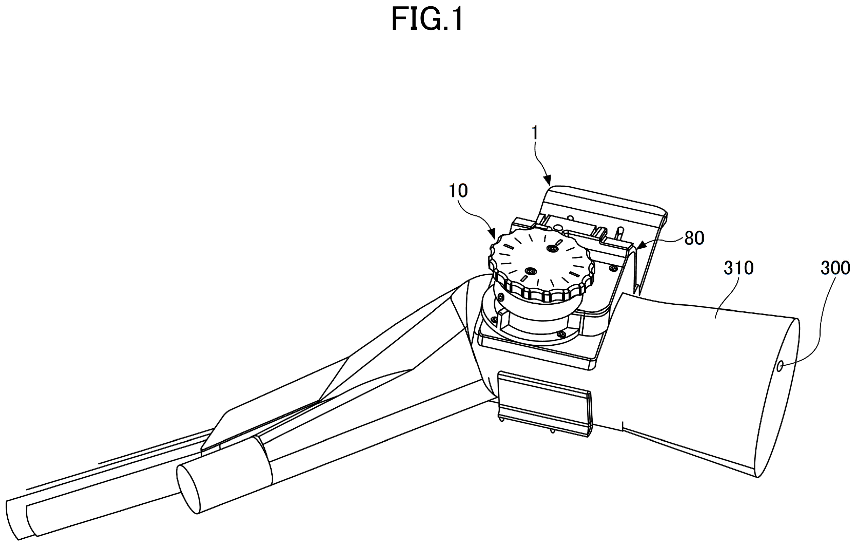

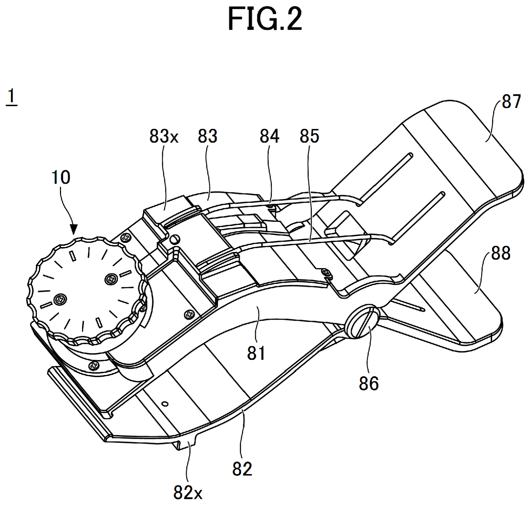

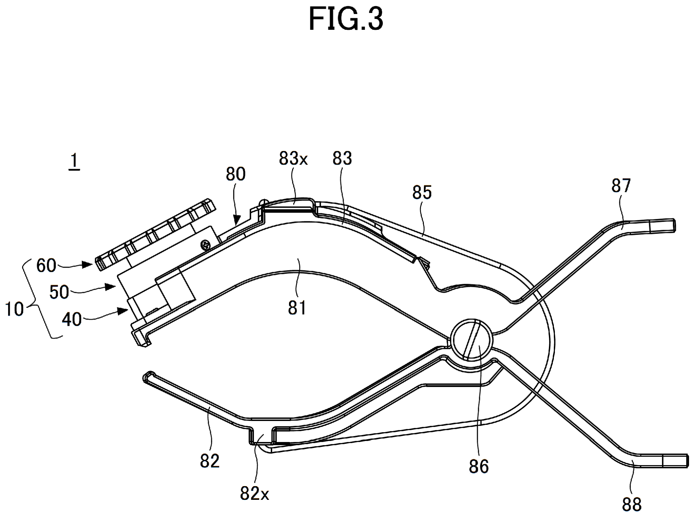

FIG. 1 is an oblique view (part 1) illustrating a pulse wave measuring device according to a first embodiment, and schematically showing a state in which the pulse wave measuring device is attached to a wrist of a subject. FIG. 2 is an oblique view (part 2) illustrating the pulse wave measuring device according to the first embodiment. FIG. 3 is a side view illustrating the pulse wave measuring device according to the first embodiment.

Referring to FIGS. 1 to 3, the pulse wave measuring device 1 is a wearable device that can be attached to a subject, and mainly includes a sensor part 10 and an attaching part 80.

As shown in FIG. 1, the pulse wave measuring device 1 is attached to a wrist of a subject via the attaching part 80 such that, for example, a pulse wave sensor 20 (described later) including a strain generating body 22 is in contact with skin 310 of a subject above a radial artery 300. A pulse wave is a waveform of a volume change of a blood vessel caused as the heart pumps out blood, and the pulse wave measuring device 1 can monitor the volume change of the blood vessel.

The sensor part 10 is a part including a pulse wave sensor for detecting a pulse wave of a subject. The sensor part 10 includes a movable part 30, a fixed part 40, a connecting part 50, and a rotating part 60. Details of the sensor part 10 will be described later.

The attaching part 80 is connected outside the sensor part 10, and is attachable to a subject. The attaching part 80 includes a first curved member 81 and a second curved member 82, which are curved in opposite directions and face each other, to be attachable to a wrist of a subject. A cap part 83 is provided on the first curved member 81. The first curved member 81, the second curved member 82 and the cap part 83 may be composed of, for example, resin and the like.

The first curved member 81 and the second curved member 82 are biased by biasing members 84 and 85 bent in an approximately letter-V shape, and are supported on a swing shaft 86 to be able to shift between a closed state and an opened state. In the illustrated example, one end of each of the biasing members 84 and 85 is attached to a spring attaching part 83x provided on the cap part 83, and the other end thereof is attached to a spring attaching part 82x provided on the second curved member 82. The biasing members 84 and 85 may be composed of, for example, metal and the like. The biasing members 84 and 85 are, for example, torsion springs, but may be leaf springs and the like.

The sensor 10 is disposed at one end of the first curved member 81 in the longitudinal direction of the first curved member 81, and the swing shaft 86 is disposed at the other end of the first curved member 81 in the longitudinal direction of the first curved member 81. The sensor 10 is attached to the first curved member 81 by, for example, a screw or the like. The swing shaft 86 may be formed integrally with the first curved member 81, or may be composed of a separate body that is joined to the first curved member 81.

A first operation part 87 is provided on a side of the first curved member 81 that is opposite to the sensor part 10 across the swing shaft 86. A second operation part 88 is provided on a side of the second curved member 82 that is opposite to the sensor part 10 across the swing shaft 86. The first operation part 87 and the second operation part 88 may be composed of, for example, resin. The first operation part 87 may be formed integrally with the first curved member 81, or may be composed of a separate body that is joined to the first curved member 81. The second operation part 88 may be formed integrally with the second curved member 82, or may be composed of a separate body that is joined to the second curved member 82.

When attaching the pulse wave measuring device 1 to a subject, the subject or a caregiver or the like who takes care of the subject pinches the first operation part 87 and the second operation part 88 to bring them close to each other. Thus, the sensor part 10 sides of the first curved member 81 and the second curved member 82 are opened, and the pulse wave measuring device 1 can be attached to the subject.

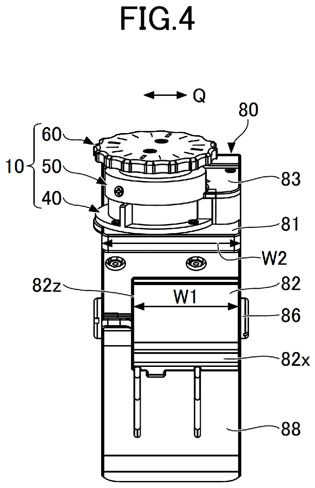

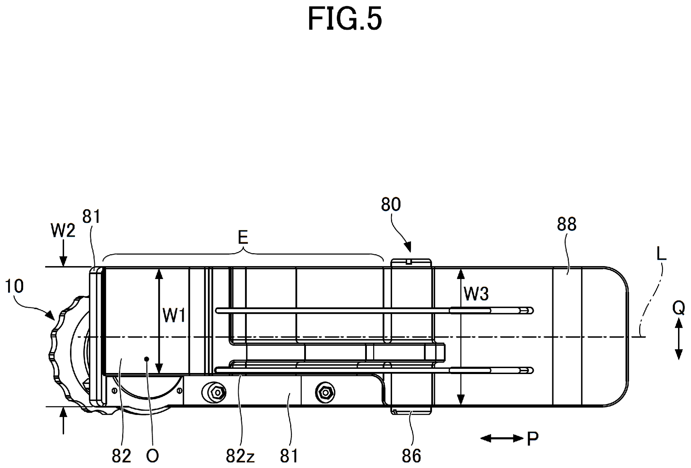

FIG. 4 is a front view illustrating the pulse wave measuring device according to the first embodiment. FIG. 5 is a bottom view illustrating the pulse wave measuring device according to the first embodiment. In FIGS. 4 and 5, Q denotes the transverse direction of the first curved member 81 and the second curved member 82. In FIG. 5, P denotes the longitudinal direction of the first curved member 81 and the second curved member 82.

The second curved member 82 includes a region E of which a width W1 in the transverse direction Q is narrower than a width W2 of the first curved member 81 in the transverse direction Q as shown in FIGS. 4 and 5. The second curved member 82 has an asymmetrical shape with respect to an imaginary line L that bisects a maximum width part of the second curved member 82 in the transverse direction Q and extends in the longitudinal direction P. The widths W1 and W2 are, for example, constant, respectively.

A width W3 of the second curved member 82 in the transverse direction Q on a side of the second curved member 82 that is farther from the sensor part 10 than the region E is in the longitudinal direction P may be, for example, the same as the width W2 of the first curved member 81 in the transverse direction Q. In this case, the first curved member 81 and the second curved member 82 can be firmly attached to the wrist of the subject.

It is preferable that the second curved member 82 has a cut-out part 82z that is obtained by cutting the second curved member 82 from one end of the second curved member 82 in the transverse direction Q toward the imaginary line L. In this case, the region E is adjacent to the cut-out part 82z in the transverse direction Q of the second curved member 82. When the second curved member 82 has the cut-out part 82z, it is easy to provide the second curved member 82 with the region E having the narrow width in the transverse direction Q. In a case of not forming the cut-out part 82z, for example, two members having different widths in the transverse direction Q can be joined in the longitudinal direction to form the second curved member 82 having the region E.

Assume a case of the pulse wave measuring device 1 in which the second curved member 82 does not include the region E of which the width in the transverse direction is narrower than the width of the first curved member 81 in the transverse direction. In this case, if a hand of the subject is curved in the direction shown in FIG. 1 when measuring a pulse wave with the pulse wave measuring device 1, the dorsum of the hand touches the second curved member 82, which inclines the sensor part 10. As a result, the strain generating body 22 of the pulse wave sensor 20 might be inclined by being unable to be kept horizontal with respect to the radial artery, and might be displaced from the position above the radial artery. As a result, the pulse wave measuring device 1 cannot accurately measure a pulse wave.

However, in the pulse wave measuring device 1, the second curved member 82 includes the region E of which the width in the transverse direction is narrower than the width of the first curved member 81 in the transverse direction. The pulse wave measuring device 1 is attached to a wrist of a subject such that the side of the pulse wave measuring device 1 on which the cut-out part 82z is provided is on the fingertip side. In this case, even if the hand of the subject is curved in the direction shown in FIG. 1 when measuring a pulse wave with the pulse wave measuring device 1, the sensor part 10 is not inclined because the dorsum of the hand hardly touches the second curved member 82. Therefore, the strain generating body 22 (described later) of the pulse wave sensor 20 can be kept horizontal with respect to the radial artery, and is not displaced from the position above the radial artery. As a result, the pulse wave measuring device 1 can accurately measure a pulse wave.

It is preferable that the cut-out part 82z is located on a side toward the sensor part 10 in the longitudinal direction P of the second curved member 82. When the cut-out part 82z is in such a position, even if a hand of a subject is curved in the direction shown in FIG. 1, the dorsum of the hand of the subject hardly touches the second curved member 82. This helps keep the strain generating body 22 horizontal with respect to the radial artery.

As shown in FIG. 5, it is preferable that the center O of the sensor part 10 is located closer to the cut-out part 82z than the imaginary line L is in the transverse direction Q of the second curved member 82 in the bottom view. This helps keep the strain generating body 22 horizontal with respect to the radial artery, even if a hand of a subject is curved in the direction shown in FIG. 1.

The width W1 of the second curved member 82 in the transverse direction Q is, for example, 35 mm, and the width W2 of the first curved member 81 in the transverse direction Q is, for example, 45 mm. In this case, the width of the cut-out part 82z in the transverse direction Q is 10 mm. The length of the cut-out part 82z in the longitudinal direction P is, for example, 90 mm. Note that these dimensions are examples and are non-limiting.

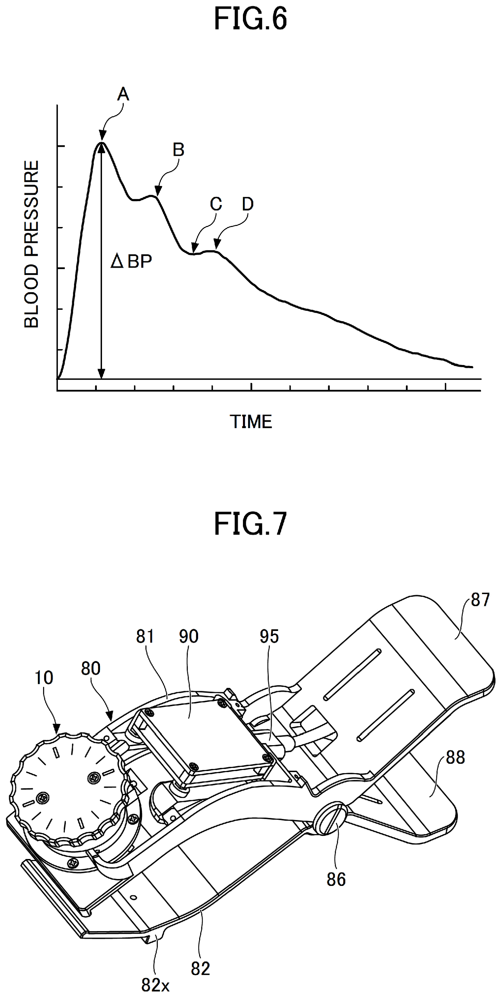

FIG. 6 is an example of a pulse wave. In FIG. 6, the vertical axis represents blood pressure, and the horizontal axis represents elapsed time measured from a minimum value of a pulse wave. In FIG. 6, A represents an ejection wave caused by blood ejection from the heart, B represents a tidal wave, which is a reflected wave from an arterial bifurcation, C represents a dicrotic notch caused by aortic valve closure, and D represents a dicrotic wave. ΔBP represents pulse pressure.

A tidal wave is generated due to reflection of an ejection wave on a peripheral artery, an arterial bifurcation, and the like while the ejection wave travels throughout the body. When a hand of a subject is curved in the direction shown in FIG. 1 and the strain generating body 22 cannot be kept horizontal with respect to the radial artery, a tidal wave peak occurs earlier than when the strain generating body 22 can be kept horizontal with respect to the radial artery. This results in a measurement error of a pulse wave.

Use of the pulse wave measuring device 1 can ensure that the strain generating body 22 is kept horizontal with respect to the radial artery even if a hand of a subject is curved in the direction shown in FIG. 1. Thus, a tidal wave peak can be accurately measured.

FIG. 7 is an oblique view for explaining the first curved member, and shows a state in which the cap part 83 is removed from the pulse wave measuring device 1. As shown in FIG. 7, the first curved member 81 may have a wiring board 90 disposed between one end side and the other end side of the first curved member 81 in the longitudinal direction. The wiring board 90 may be disposed in a gap between the first curved member 81 and the cap part 83. The wiring board 90 is electrically connected to the pulse wave sensor 20 (described later).

For example, a component contributing to the detection of a pulse wave is disposed on the wiring board 90. Examples of the component include an amplifier circuit for amplifying an output from a strain gauge 100, an AD converter for converting an output from the amplifier circuit into a digital signal, a signal processing semiconductor for processing a digital signal, and a wireless communication semiconductor for transmitting a signal processing result to the outside. A cable 95 may be connected to the wiring board 90. The cable 95 may be used for supplying power or for receiving/outputting electrical signals from/to an external circuit.

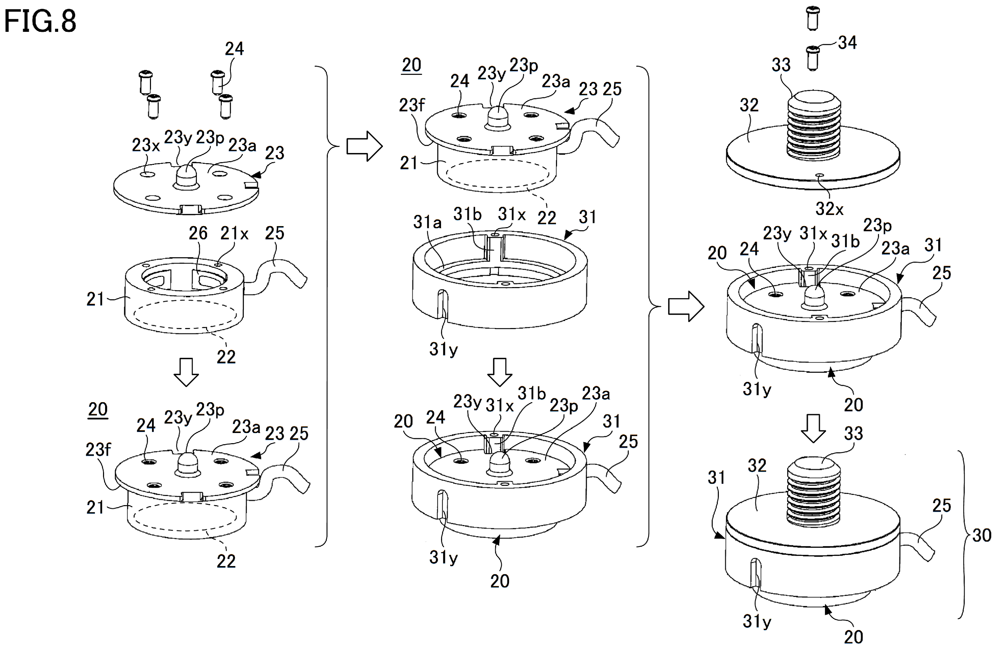

FIG. 8 is an exploded oblique view illustrating the movable part. As shown in FIG. 8, the movable part 30 includes the pulse wave sensor 20, a sensor holding part 31, a cap part 32, and an external screw part 33. The sensor holding part 31, the cap part 32, and the external screw part 33 may be composed of, for example, metal, resin, and the like.

The pulse wave sensor 20 includes, for example, a housing 21, the strain generating body 22 provided on one side of the housing 21, and a facing part 23 provided on the other side of the housing 21. The housing 21 is, for example, a cylindrical member having both ends that are open. The housing 21 may be composed of, for example, metal, resin, and the like.

The strain generating body 22 has an approximately disk shape, and is fixed to the housing 21 by an adhesive or the like to close the one side of the housing 21. As will be described later, the strain generating body 22 is a part on which, for example, a strain gauge is disposed to detect a pulse wave. A detailed structural example of the pulse wave sensor 20 including the strain generating body 22 will be described later.

The facing part 23 has an approximately disk shape, and is fixed to the housing 21 to close the other side of the housing 21. The facing part 23 includes, for example, through-holes 23x and is threadedly engaged with grooves 21x provided in the housing 21 via screws 24 inserted into the through-hole 23x. The facing part 23 faces the strain generating body 22 through a space inside the housing 21. Surfaces of the facing part 23 and the strain generating body 22 facing each other are, for example, parallel.

The facing part 23 has a first surface 23a that is a surface opposite to the surface facing the strain generating body 22. The first surface 23a of the facing part 23 is, for example, a flat surface. A pivot part 23p projecting to a side opposite to the strain generating body 22 is provided in approximately the center of the first surface 23a of the facing part 23. The pivot part 23p is, for example, an approximately circular columnar member. The center axis of the pivot part 23p is, for example, perpendicular to the first surface 23a of the facing part 23.

The facing part 23 has, for example, a flange part 23f projecting to a radially outer side of the housing 21 from the outer surface of the housing 21. The flange part 23f has, for example, a ring shape. The facing part 23 may have one or a plurality of notch parts 23y that can be used for rotation prevention and the like. The notch parts 23y are, for example, recessed from the outer periphery of the facing part 23 toward the center of the facing part 23.

The facing part 23 may be composed of, for example, metal, resin, and the like. The pivot part 23p may be formed integrally with another part of the facing part 23, or may be composed of a separate body that is joined to the another part.

The pulse wave sensor 20 includes a wire 25 for inputting and outputting electrical signals between the inside and outside of the housing 21. A plurality of mutually insulated wires may be situated inside the wire 25. One end of the plurality of wires is electrically connected to electrodes of a strain gauge described later. The pulse wave sensor 20 may have a flexible substrate or the like instead of the wire 25.

Further, one or a plurality of wiring boards 26 may be fixed to the inner surface of the housing 21, and a pair of electrodes of a strain gauge may be electrically connected to the wiring boards 26 by thin wires. Then, the wiring boards 26 may be electrically connected to the wire 25. With this structure, the forces from the wire 25 are hardly transmitted to the strain generating body 22, and the detection accuracy of the pulse wave can be improved.

The sensor holding part 31 is, for example, a cylindrical member having both ends that are open. The inner surface of the sensor holding part 31 has, for example, a stepped surface 31a projecting toward the center axis side (the center side of the sensor part 10). The stepped surface 31a is, for example, perpendicular to the axial direction of the sensor holding part 31. The stepped surface 31a has, for example, a ring shape.

The sensor holding part 31 has a positioning part 31b provided on the upper side of the stepped surface 31a and projecting from the inner surface of the sensor holding part 31 toward the center axis side. In the illustrated example, two positioning parts 31b are provided to face each other. Each positioning part 31b is provided with a groove 31x. There may be one or more positioning parts 31b.

The pulse wave sensor 20 is held inside the sensor holding part 31 with the notch parts 23y aligned with the positioning parts 31b, and is prevented from rotating relative to the sensor holding part 31. However, the pulse wave sensor 20 is not fixed to the sensor holding part 31, and has a gap that allows it to move relative to the sensor holding part 31. When the pulse wave measuring device 1 is not attached to a subject, the flange part 23f is in contact with the stepped surface 31a.

The sensor holding part 31 has a groove 31y recessed from the outer surface of the sensor holding part 31 toward the center of the sensor holding part 31. The groove 31y has an elongated shape where the longitudinal direction of the groove 31y is aligned with the axial direction of the sensor holding part 31. The groove 31y is provided to stop short of the upper end of the outer surface of the sensor holding part 31 but to reach the lower end. For example, two grooves 31y can be disposed to face each other across the center of the sensor holding part 31 in a plan view. One or more grooves 31y may be provided.

The cap part 32 has through-holes 32x. For example, the cap part 32 is threadedly engaged with the grooves 31x provided in the positioning parts 31b by screws 34 inserted into the through-holes 32x, and is fixed on the other end side of the sensor holding part 31 in the axial direction of the sensor holding part 31. The external screw part 33 is provided in the center of the upper surface of the cap part 32, and projects from the upper surface of the cap part 32 in a direction opposite to the strain generating body 22. The external screw part 33 may be formed integrally with the cap part 32, or may be composed of a separate body that is joined to the cap part 32.

In this way, the pulse wave sensor 20 is held inside the sensor holding part 31 such that the strain generating body 22 is exposed from one end side of the sensor holding part 31 in the axial direction of the sensor holding part 31. The cap part 32 is fixed to the other end side of the sensor holding part 31 in the axial direction of the sensor holding part 31.

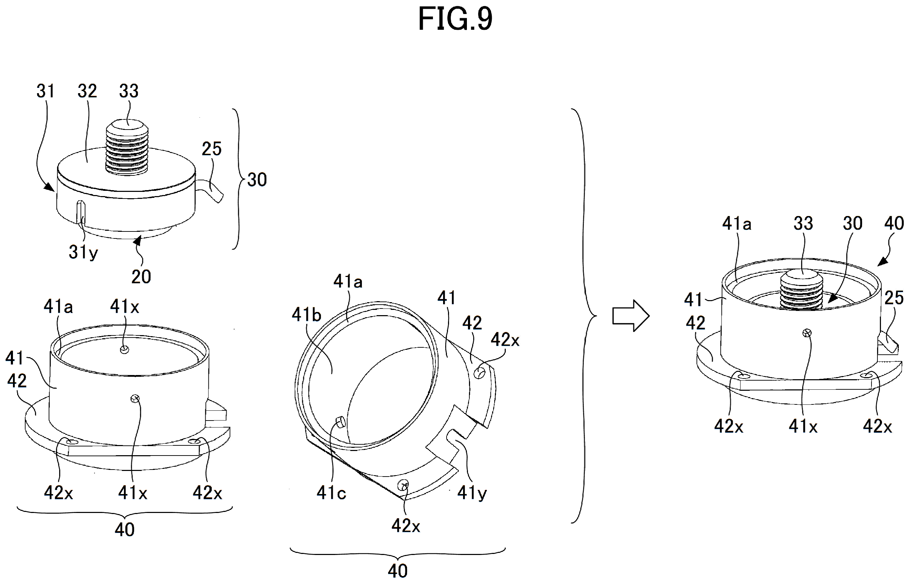

FIG. 9 is an exploded oblique view illustrating the fixed part and the movable part. In FIG. 9, for convenience, two views of the fixed part 40 viewed from different perspectives are shown.

The fixed part 40 has a cylindrical part 41 and a flange part 42. The cylindrical part 41 is, for example, a cylindrical member having both ends that are open. The flange part 42 is a member projecting to the radially outward side of the cylindrical part 41 from at least a part of the outer surface of the cylindrical part 41. The flange part 42 may have a notch. The fixed part 40 may be composed of, for example, metal, resin, and the like. The flange part 42 may be formed integrally with the cylindrical part 41 or may be composed of a separated body that is joined to the cylindrical part 41.

The cylindrical part 41 has, for example, a stepped surface 41a projecting toward the center axis side (the central side of the cylindrical part 41). The stepped surface 41a is, for example, perpendicular to the axial direction of the cylindrical part 41. The stepped surface 41a has, for example, a ring shape. The cylindrical part 41 has two projections 41c projecting from an inner surface 41b toward the center axis side. The projections 41c have, for example, a circular columnar shape. For example, the two projections 41c may be arranged to face each other across the center of the cylindrical part 41 in a plan view. One or more projections 41c may be provided.

The cylindrical part 41 has through-holes 41x penetrating from the outer surface of the cylindrical part 41 to the inner surface of the cylindrical part 41. For example, two through-holes 41x may be arranged to face each other across the center of the cylindrical part 41 in a plan view. The cylindrical part 41 may have a notch part 41y through which the wire 25 is passed. The flange part 42 has through-holes 42x. For example, four through-holes 42x can be arranged at positions symmetrical to one another across the center of the cylindrical part 41 in a plan view.

The movable part 30 is accommodated inside the cylindrical part 41 of the fixed part 40 such that the two grooves 31y are fitted with the two projections 41c. The movable part 30 is movable in the axial direction of the cylindrical part 41. Since the projections 41c are fitted with the grooves 31y, the movable part 30 is hardly movable in the circumferential direction of the cylindrical part 41. That is, the projections 41c fitted with the grooves 31y prevent the sensor holding part 31 from rotating relative to the cylindrical part 41. Further, the downward moving range of the movable part 30 is restricted to a range in which the projections 41c can be fitted with the grooves 31y, that is, a range defined by the length of the grooves 31y in the longitudinal direction.

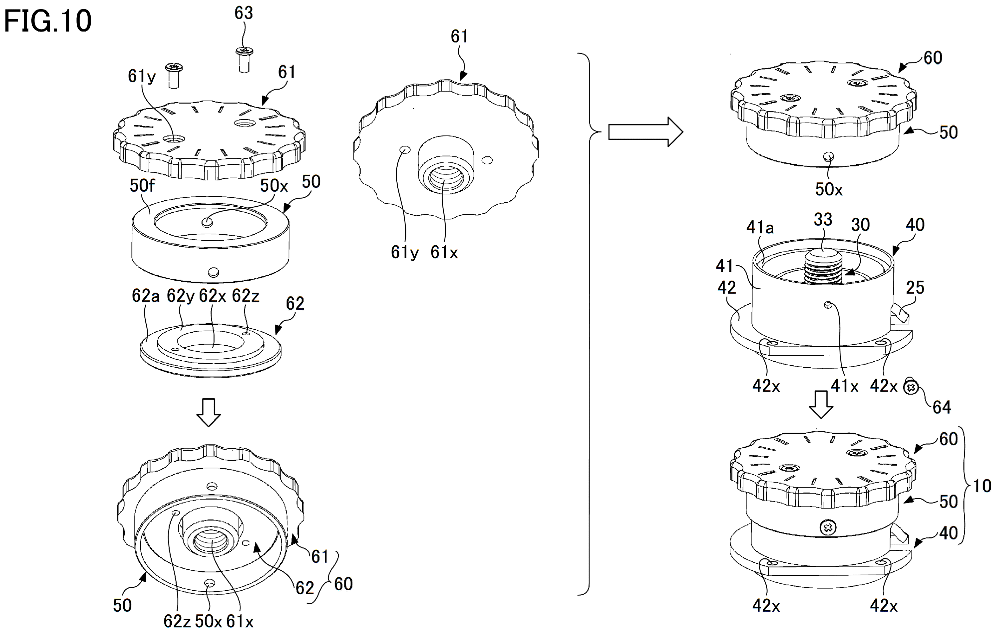

FIG. 10 is an exploded oblique view illustrating the sensor part. As shown in FIG. 10, the sensor part 10 includes the movable part 30, the fixed part 40, the connecting part 50, and the rotating part 60. The connecting part 50 and the rotating part 60 can be composed of, for example, metal, resin, and the like. In FIG. 10, two views of a first disk part 61 viewed from different perspectives are shown for convenience.

The rotating part 60 closes one opening of the cylindrical part 41 and rotates with respect to the fixed part 40 about the center axis of the cylindrical part 41 as the rotation axis. The strain generating body 22 is exposed from the other opening of the cylindrical part 41.

The rotating part 60 includes: the first disk part 61 including, in the center of the first disk part 61, an internal screw part 61x projecting toward the strain generating body 22 side; and a second disk part 62 having a diameter smaller than that of the first disk part 61. A jagged structure is formed on the outer surface of the first disk part 61 such that, for example, the rotating part 60 is not slippery when a subject or the like rotates the rotating part 60. The internal screw part 61x is a cylindrical member screw-cut in the inner surface of the cylindrical member. The first disk part 61 has through-holes 61y.

The second disk part 62 has a through-hole 62x in the center of the second disk part 62. The second disk part 62 has a ring-shaped projection 62y provided to surround the through-hole 62x in a plan view, and a ring-shaped stepped surface 62a is on the outer side of the projection 62y. The stepped surface 62a is, for example, perpendicular to the axial direction of the second disk part 62. The second disk part 62 has through-holes 62z.

The connecting part 50 is, for example, a cylindrical member having both ends that are open. The connecting part 50 has, on an end of the connecting part 50 on the first disk part 61 side, a ring-shaped flange part 50f projecting radially inward from the outer surface of the connecting part 50. The connecting part 50 has through-holes 50x in the side surface. For example, two through-holes 50x can be arranged to face each other across the center of the connecting part 50 in a plan view.

The second disk part 62 is accommodated in the connecting part 50 and fixed to the first disk part 61 with the flange part 50f sandwiched between the second disk part 62 and the first disk part 61. Specifically, the first disk part 61 and the second disk part 62 are threadedly engaged with each other by screws 63 with the flange part 50f of the connecting part 50 sandwiched between the lower surface of the first disk part 61 and the stepped surface 62a of the second disk part 62. With this structure, the connecting part 50 does not slip out from the rotating part 60 composed of the first disk part 61 and the second disk part 62. The rotating part 60 is rotatable relative to the connecting part 50.

The structure including the connecting part 50 and the rotating part 60 is set on the fixed part 40. Here, the internal screw part 61x of the first disk part 61 and the external screw part 33 of the movable part 30 are threadedly engaged with each other in a rotatable manner. Thus, the cap part 32 is connected to the rotating part 60. Then, the through-holes 41x of the fixed part 40 and the through-holes 50x of the connecting part 50 are aligned in position and threadedly engaged with each other by screws 64, and the connecting part 50 is fixed to the cylindrical part 41. Thus, the rotating part 60 is rotatable with respect to the fixed part 40.

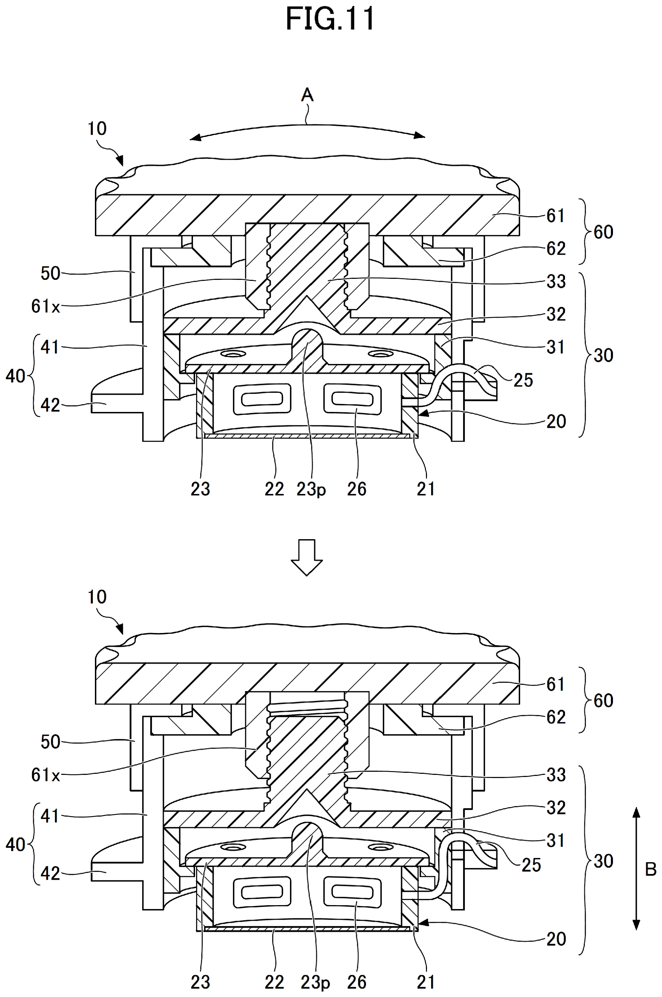

FIG. 11 is a cross-sectional view illustrating the sensor part. As shown in FIG. 11, the movable part 30 does not rotate with respect to the fixed part 40, and reciprocates in the axial direction of the cylindrical part 41 as the rotating part 60 rotates. That is, when the rotating part 60 including the first disk part 61 and the second disk part 62 rotates in the direction of an arrow A, the threadedly engaged state of the internal screw part 61x and the external screw part 33 changes, and the movable part 30 including the pulse wave sensor 20 reciprocates in the axial direction of the cylindrical part 41 (the direction of an arrow B). The movable part 30 can be configured to move from a position at which the strain generating body 22 projects from the lower end of the fixed part 40 to a position above the lower end of the fixed part 40.

With such a structure, the position of the movable part 30 can be adjusted such that the strain generating body 22 is positioned above the lower end of the fixed part 40 when the pulse wave measuring device 1 is not attached to a subject. As a result, since the strain generating body 22 would not be touched by the subject when attaching the pulse wave measuring device 1 to the subject, plastic deformation of the strain generating body 22 can be inhibited.

Further, after the pulse wave measuring device 1 is attached to the subject, the rotating part 60 is rotated to change the position of the movable part 30, such that the radial artery of the subject can be pressed appropriately. Here, for example, the rotating part 60 may be rotated while monitoring a pulse wave signal obtained from the pulse wave sensor 20, such that the amplitude of the pulse wave signal may be adjusted to be large as possible. Alternatively, a sound signal or an optical signal informing that the amplitude of the pulse wave signal has entered a predetermined range may be generated to inform the subject of the optimally adjusted position of the movable part 30.

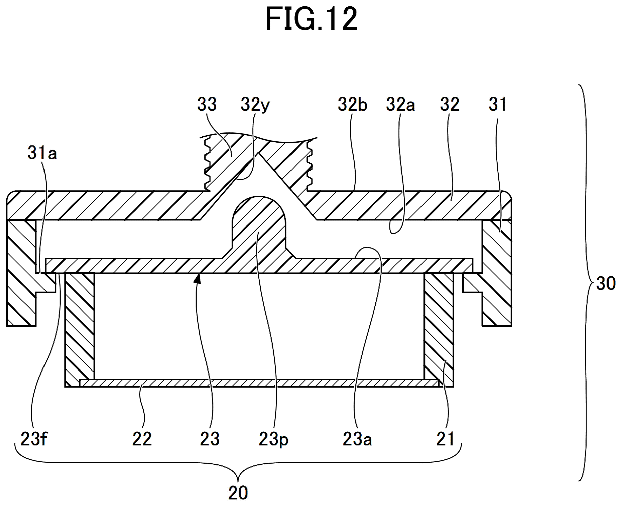

FIG. 12 is a cross-sectional view (part 1) for explaining the pivot part. As shown in FIG. 12, the cap part 32 has a first surface 32a and a second surface 32b opposite to the first surface 32a. The first surface 32a and the second surface 32b are, for example, parallel. A recessed portion 32y opening toward the first surface 32a side is provided approximately in the center of the first surface 32a of the cap part 32. The inner surface of the recessed portion 32y is, for example, one curved surface inclined from the axial direction of the external screw part 33. In the cross-sectional view, the inner surface of the recessed portion 32y may be partially or entirely curved.

The recessed portion 32y has, for example, a conical or frusto-conical shape. The recessed portion 32y may have a circular-columnar shape having a diameter larger than that of the pivot part 23p. From the viewpoint of reducing play between the pivot part 23p and the recessed portion 32y to facilitate centering, it is preferable that the inner surface of the recessed portion 32y is a single curved surface, such as that of a conical shape, a frusto-conical shape, and the like, that is inclined with respect to the axial direction of the sensor part 10.

The recessed portion 32y may be disposed at a position coinciding with the external screw part 33 when viewed in the axial direction of the movable part 30 (the vertical direction in FIG. 12), and a part of the recessed portion 32y may be disposed in the external screw part 33. In this way, the cap part 32 can be partially reduced in thickness.

The first surface 23a of the facing part 23 and the first surface 32a of the cap part 32 face each other. In the facing part 23, the distal end side (the recessed portion 32y side) of the pivot part 23p has, for example, a dome shape. Here, the dome shape is a shape in which the height from the first surface 23a is the maximum near the center axis and decreases toward the periphery. The distal end side of the pivot part 23p may be a part of a spherical surface or a part of an aspherical surface.

The flange part 23f of the facing part 23 is disposed between the stepped surface 31a of the sensor holding part 31 and the first surface 32a of the cap part 32. With such a structure, the flange part 23f serves as a stopper for preventing the pulse wave sensor 20 from slipping out from the sensor holding part 31 downward.

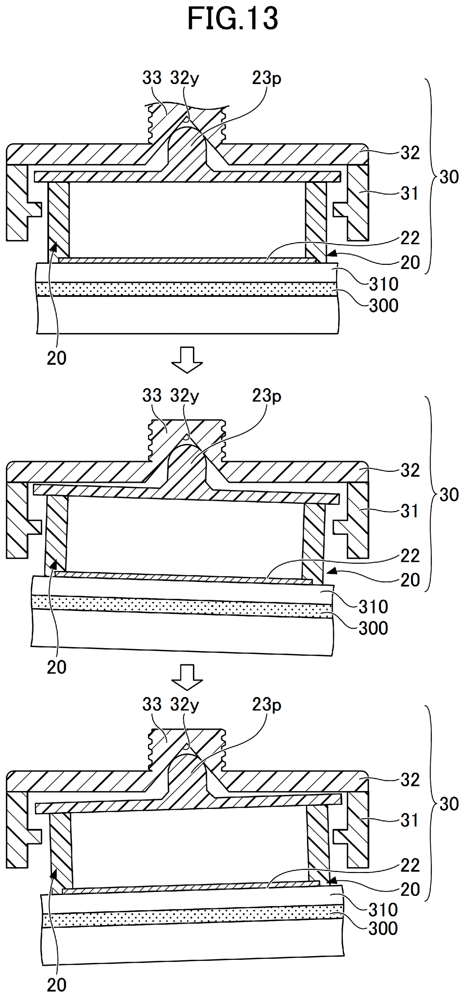

FIG. 13 is a cross-sectional view (part 2) for explaining the pivot part. In any view included in FIG. 13, the attaching part 80 of the pulse wave measuring device 1 is attached to a subject. The radial artery of the subject is schematically shown at the reference numeral 300. When the attaching part 80 is attached to the subject, as shown in FIG. 13, the strain generating body 22 of the pulse wave sensor 20 is brought into contact with the skin 310 of the subject above the radial artery 300, and the pulse wave sensor 20 is pushed up toward the cap part 32, whereby the pivot part 23p and the recessed portion 32y come into contact with each other. The pivot part 23p and the recessed portion 32y come into, for example, linear contact with each other. In this way, the pivot part 23p and the recessed portion 32y can be easily centered with reduced play. However, this is a non-limiting feature, and the pivot part 23p and the recessed portion 32y may come into point contact or surface contact with each other.

As described above, when the attaching part 80 is not attached to the subject, the pulse wave measuring device 1 is in a first state in which the pivot part 23p and the recessed portion 32y are not in contact with each other (see FIG. 12). When the attaching part 80 is attached to the subject, it switches to a second state in which the pivot part 23p and the recessed portion 32y are in contact with each other (see FIG. 13).

As shown in the upper, middle, and lower parts of FIG. 13, when the pivot part 23p and the recessed portion 32y come into contact with each other, the pulse wave sensor 20 becomes swingable in any of the directions defined in the range of 360 degrees, on a fulcrum, which is the contact part at which the pivot part 23p and the recessed portion 32y contact each other. That is, the detection surface of the strain generating body 22 of the pulse wave sensor 20 can be inclined at any angle along the radial artery 300 of the subject.

Thus, since the angle of the detection surface of the strain generating body 22 of the pulse wave sensor 20 can follow the radial artery 300 of the subject, the detection surface of the strain generating body 22 can contact the radial artery 300 of the subject at an appropriate angle. That is, the detection surface of the strain generating body 22 of the pulse wave sensor 20 can be placed approximately in parallel with the radial artery 300 of the subject. As a result, a weak pulse wave signal can be detected, and the pulse wave measurement accuracy can be improved.

Further, it is possible to place the pulse wave sensor 20 on the radial artery easily, by operating the first operation part 87 and the second operation part 88, to shift the pulse wave measuring device 1 between the opened state and the closed state a plurality of times and finely adjust the position of the pulse wave sensor 20. Here, it is preferable to monitor an output signal from the pulse wave sensor 20 and find a position where the amplitude of the output signal is as large as possible.

Further, in the pulse wave measuring device 1, the wiring board 90 is provided in an empty space in the first curved member 81, and components contributing to the detection of a pulse wave are installed on the wiring board 90, thereby realizing a small-sized pulse wave measuring device that is mounted with necessary components.

Further, since the pulse wave measuring device 1 is provided with the biasing members 84 and 85, it is possible to press the radial artery of the subject moderately.

### [Pulse Wave Sensor]

Here, an example of a pulse wave sensor having a plurality of strain gauges will be shown as an example of the pulse wave sensor 20. Descriptions of any components of the pulse wave sensor 20 that have already been described will be omitted.

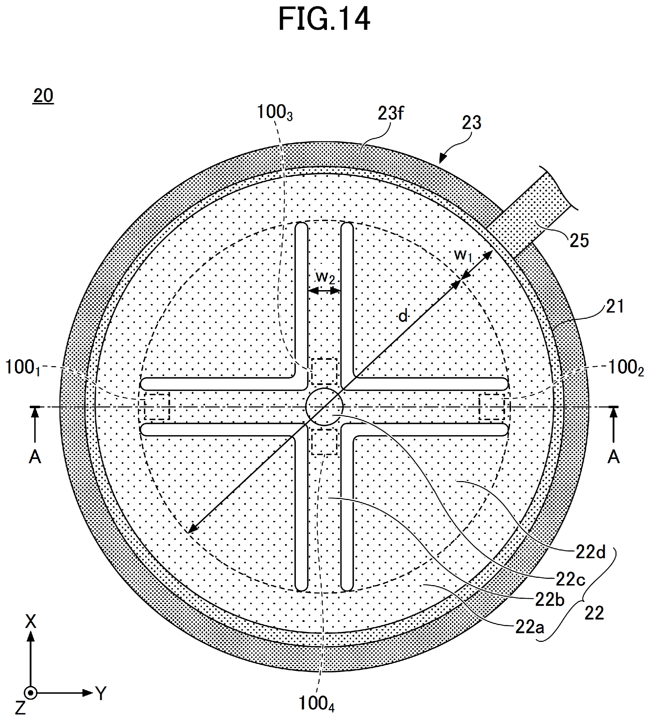

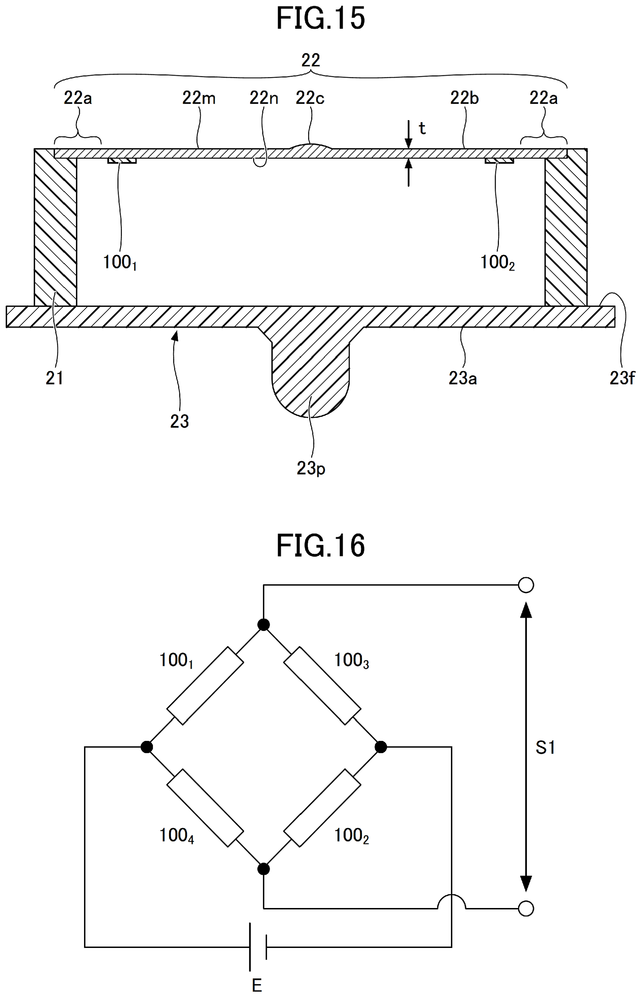

FIG. 14 is a plan view illustrating a pulse wave sensor according to a first embodiment. FIG. 15 is a cross-sectional view illustrating the pulse wave sensor according to the first embodiment, showing a cross-section along a line A-A in FIG. 14.

Referring to FIGS. 14 and 15, the pulse wave sensor 20 includes the housing 21, the strain generating body 22, the facing part 23, the wire 25, and a plurality of strain gauges (strain gauges 100₁, 100₂, 100₃, and 100₄).The facing part 23 and the wire 25 are as described above. The strain gauges 100₁, 100₂, 100₃, and 100₄ may be collectively referred to as strain gauges 100, where there is no particular need to distinguish them.

The strain generating body 22 includes a base part 22a, a beam part 22b, a load part 22c, and extension parts 22d. The strain generating body 22 has a flat plate shape. The strain generating body 22 includes a first main surface 22m and a second main surface 22n located on the side opposite to the first main surface 22m. The first main surface 22m is a surface facing the subject side.

The material of the strain generating body 22 may be, for example, metal, ceramic, glass, and the like.. Examples of the metal used as the material of the strain generating body 22 include SUS (stainless steel), copper, aluminum, and the like. The strain generating body 22 can be formed as one body by, for example, a press forming method and the like. The thickness t of the strain generating body 22 excluding the load part 22c is constant. The thickness t can be, for example, 0.03 mm or greater and 0.3 mm or less.

In the description of FIGS. 14 and 15, for convenience, a side of the pulse wave sensor 20 on which the load part 22c of the strain generating body 22 is provided is referred to as the "upper side", and a side on which the load part 22c of the strain generating body 22 is not provided is referred to as the "lower side". A surface of each part that is located on the upper side is referred to as the "upper surface", and a surface of each part that is located on the lower side is referred to as the "lower surface". However, the pulse wave sensor 20 can be used upside down. The pulse wave sensor 20 can be placed at a desired angle. Viewing in a plan view means viewing an object in a direction normal to the first main surface 22m of the strain generating body 22 from the upper side to the lower side. A planar shape means a shape of an object when the object is viewed in the normal direction.

In the pulse wave sensor 20, the housing 21 is a part for holding the strain generating body 22. The housing 21 can be composed of, for example, metal, resin, and the like.

In the strain generating body 22, the base part 22a is a circular frame-shaped (ring-shaped) region that is on the outer side of a circular broken line shown in FIG. 14. The region that is on the inner side of the circular broken line may be referred to as a circular opening. That is, the base part 22a of the strain generating body 22 has a circular opening. The width w₁ of the base part 22a is, for example, 1 mm or greater and 5 mm or less. The inner diameter d of the base part 22a (i.e., the diameter of the circular opening) is, for example, 10 mm or greater and 15 mm or less.

The beam part 22b is provided to bridge the interior of the base part 22a. The beam part 22b includes, for example, two beams intersecting each other in a cross shape in a plan view, and the region in which the two beams intersect includes the center of the circular opening. In the example of FIG. 14, one beam that is a constituent of the cross has its longitudinal direction in the X direction, and the other beam that is a constituent of the cross has its longitudinal direction in the Y direction, and both beams cross perpendicularly to each other. It is preferable that each of the two beams crossing perpendicularly to each other is located on the inner side of the inner diameter d of the base part 22a (the diameter of the circular opening) and is as long as possible. That is, it is preferable that the length of each beam is approximately equal to the diameter of the circular opening. In each of the beams that are the constituents of the beam part 22b, the width w₂ other than in the intersecting region is constant, and is, for example, 1 mm or greater and 5 mm or less. Although it is not essential that the width w₂ is constant, it is preferable that the width w₂ is constant because a strain can be linearly detected.

The load part 22c is provided on the beam part 22b. The load part 22c is provided in, for example, the region where the two beams that are the constituents of the beam part 22b intersect. The load part 22c projects from the upper surface of the beam part 22b. The amount of projection of the load part 22c with respect to the upper surface of the beam part 22b is, for example, approximately 0.1 mm. The beam part 22b is flexible and deforms elastically when a load is applied to the load part 22c. The upper surface of the beam part 22b is a part of the first main surface 22m of the strain generating body 22.

The four extension parts 22d are fan-shaped parts extending in the direction to the beam part 22b from the inner side of the base part 22a in a plan view. A gap of approximately 1 mm is provided between the respective extension parts 22d and the beam part 22b. Since the extension parts 22d do not contribute to the sensing of the pulse wave sensor 20, they do not need to be provided.

An output signal from the pulse wave sensor 20 is generated based on outputs from the plurality of strain gauges. In the illustrated example, the pulse wave sensor 20 includes a pair of strain gauges 100₁ and 100₂ arranged to face each other across the load part 22c in a plan view on the beam extending in the Y direction on the second main surface 22n of the strain generating body 22. Further the pulse wave sensor 20 includes another pair of strain gauges 100₃ and 100₄ arranged to face each other across the load part 22c in a plan view on the beam extending in the X direction intersecting the beam on which the pair of strain gauges 100₁ and 100₂ are arranged.

On the beam extending in the Y direction, the strain gauges 100₁ and 100₂ detect a compressive strain of the strain generating body 22 caused when the load part 22c is pressed. On the beam extending in the X direction, the strain gauges 100₃ and 100₄ detect a tensile strain of the strain generating body 22 caused when the load part 22c is pressed. The spacing between the strain gauges 100₁ and 100₂ for detecting a compressive strain is wider than the spacing between the strain gauges 100₃ and 100₄ for detecting a tensile strain. By arranging the strain gauges 100 in this manner, it is possible to effectively detect a compressive strain and a tensile strain and obtain a high output by means of a bridge circuit constituting a full bridge.

The strain gauges 100₁ to 100₄ are connected to constitute respective sides of one bridge circuit, and an output signal (a signal indicating a pulse wave) from the pulse wave sensor 20 can be generated by the bridge circuit. FIG. 16 is an example of a bridge circuit. In the bridge circuit shown in FIG. 16, the strain gauge 100₁ constitutes one side on the upper left. The strain gauge 100₂ constitutes one side on the lower right. The strain gauge 100₃ constitutes one side on the upper right. The strain gauge 100₄ constitutes one side on the lower left.

In FIG. 16, a direct-current (DC) voltage E is supplied between a connecting part at which the upper left side and the lower left side are connected and a connecting part at which the upper right side and the lower right side are connected. Thus, an analog voltage output signal S1 can be obtained from between a connecting part at which the upper left side and the upper right side are connected and a connecting part at which the lower left side and the lower right side are connected. For example, the wiring pattern constituting the bridge circuit can be provided on the wiring board 90.

In the pulse wave sensor 20, when the load part 22c contacts the radial artery of the subject, a load is applied to the load part 22c in accordance with a pulse wave of the subject, and the beam part 22b elastically deforms, thereby changing the resistance values of the resistors of the strain gauges 100. The pulse wave sensor 20 can detect the pulse wave based on the changes in the resistance values of the resistors of the strain gauges 100 caused by the deformation of the beam part 22b. The pulse wave is detected from the bridge circuit in the form of an output signal S1 as a periodic voltage change.

In the foregoing description, an example in which the pulse wave sensor 20 includes four strain gauges and generates the output signal S1 by connecting the four strain gauges as a full bridge has been shown. However, the pulse wave sensor 20 may include two strain gauges and generate the output signal S1 by connecting the two strain gauges as a half bridge.

### [Strain Gauge 100]

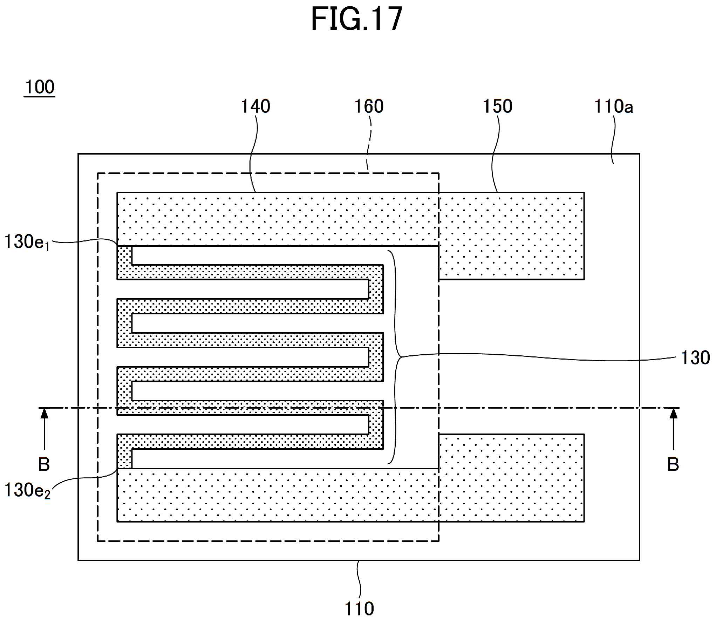

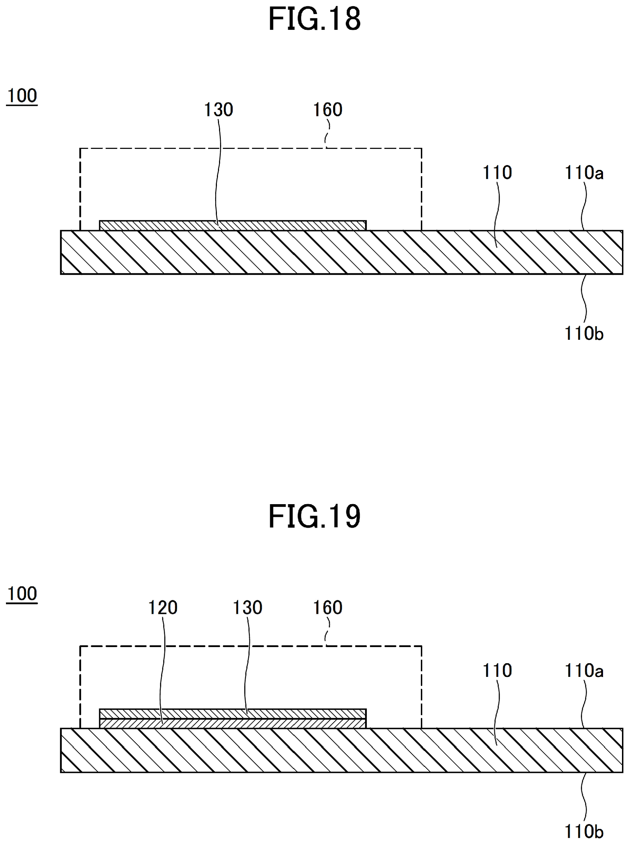

FIG. 17 is a plan view illustrating a strain gauge according to the first embodiment. FIG. 18 is a cross-sectional view (part 1) illustrating the strain gauge according to the first embodiment, and shows a cross-section along line B-B in FIG. 17.

Referring to FIGS. 17 and 18, the strain gauge 100 includes a substrate 110, a resistor 130, a wiring 140, electrodes 150, and a cover layer 160. That is, the strain gauge 100 includes the resistor 130 as a detection element. The cover layer 160 can be provided on as-needed basis. In FIGS. 17 and 18, only the outer edges of the cover layer 160 are shown by a broken line for convenience. First, each component of the strain gauge 100 will be described in detail.

In the description of the strain gauge with reference to FIGS. 17 to 19, the definitions of the upper and lower surfaces are different from those in the other drawings. Specifically, in FIGS. 17 to 19, a side of the strain gauge 100 on which the resistor 130 of the substrate 110 is provided is referred to as the "upper side" and a side of the strain gauge 100 on which the resistor 130 is not provided is referred to as the "lower side" for convenience. Further, a surface of each part located on the upper side is referred to as the "upper surface", and a surface of each part located on the lower side is referred to as the "lower surface". However, the strain gauge 100 can be used upside down. The strain gauge 100 can also be placed at a desired angle. Viewing in a plan view means viewing an object in a direction normal to the upper surface 110a of the substrate 110 from the upper side to the lower side. A planar shape means the shape of an object when viewed in the normal direction. The strain gauge 100 is attached to the second main surface 22n of the strain generating body 22 such that the substrate 110 faces the second main surface 22n side of the strain generating body 22.

The substrate 110 is a member serving as a base layer on which the resistor 130 and the like are formed. The substrate 110 is flexible. The thickness of the substrate 110 is not particularly limited and may be appropriately determined in accordance with the purpose of use of the strain gauge 100 and the like. For example, the thickness of the substrate 110 may be approximately 5 µm to 500 µm. The thickness of the substrate 110 is preferably within the range of 5 µm to 200 µm from the viewpoint of the transmissibility of a strain from the second main surface 22n of the strain generating body 22 to the sensitive part and dimensional stability against environmental changes. The thickness of the substrate 110 is preferably 10 µm or greater from the viewpoint of insulation performance.

The substrate 110 may be formed from, for example, an insulating resin film, such as a polyimide (PI) resin, an epoxy resin, a polyetheretherketone (PEEK) resin, a polyethylene naphthalate (PEN) resin, a polyethylene terephthalate (PET) resin, a polyphenylene sulfide (PPS) resin, a liquid crystal polymer (LCP) resin, a polyolefin resin, and the like. The film refers to a member having a thickness of approximately 500 µm or less and having flexibility.

When the substrate 110 is formed from an insulating resin film, the insulating resin film may contain a filler, an impurity, or the like. The substrate 110 may be formed from, for example, an insulating resin film containing a filler, such as silica, alumina, or the like.

Examples of the material of the substrate 110 other than the resin include crystalline materials, such as SiO₂, ZrO₂ (including YSZ), Si, Si₂N₃, Al₂O₃ (including sapphire), ZnO, and perovskite ceramics (CaTiO₃, BaTiO₃), and the like. In addition to the aforementioned crystalline materials, amorphous glass or the like may be used as a material of the substrate 110. Metal, such as aluminum, aluminum alloy (duralumin), titanium, and the like, may be used as the material of the substrate 110. When a metal substrate 110 is used, an insulating film is provided to cover the upper surface 110a.

The resistor 130 is a thin film formed in a predetermined pattern on the upper side of the substrate 110. In the strain gauge 100, the resistor 130 is a sensitive part that generates a resistance change upon receiving a strain. The resistor 130 may be directly formed on the upper surface 110a of the substrate 110, or may be formed on the upper surface 110a of the substrate 110 via another layer. In FIG. 17, for convenience, the resistor 130 is shown with a dense stippled pattern.

The resistor 130 has a structure in which a plurality of thin and long portions are arranged at predetermined intervals with their longitudinal direction set in the same direction (the direction of the line B-B in the example of FIG. 17), and the end portions of adjacent thin and long portions are connected on alternate sides, to have a zigzag-folded-back shape as a whole. The longitudinal direction of the plurality of thin and long portions is a grid direction, and the direction perpendicular to the grid direction is a grid width direction (the direction perpendicular to the line B-B in the example of FIG. 17).

End portions, on one side in the longitudinal direction, of two thin and long portions positioned on the outermost sides in the grid width direction are bent in the grid width direction to form respective terminals 130e₁ and 130e₂ of the resistor 130 in the grid width direction. The respective terminals 130e₁ and 130e₂ of the resistor 130 in the grid width direction are electrically connected to the electrodes 150 via the wiring 140. In other words, the wiring 140 electrically connect the terminals 130e₁ and 130e₂ of the resistor 130 in the grid width direction to the electrodes 150, respectively.

The resistor 130 can be formed, for example, from a material containing chromium (Cr), a material containing nickel (Ni), or a material containing both Cr and Ni. That is, the resistor 130 can be formed from a material containing at least one of Cr or Ni. Examples of the material containing Cr include a Cr mixed phase film. Examples of the material containing Ni include copper nickel (Cu-Ni). Examples of the material containing both Cr and Ni include nickel chromium (Ni-Cr).

Here, the Cr mixed phase film is a film in which Cr, CrN, Cr₂N, and the like are mixed. The Cr mixed phase film may contain unavoidable impurities, such as chromium oxide and the like.

The thickness of the resistor 130 is not particularly limited, and may be appropriately determined in accordance with the purpose of use of the strain gauge 100 and the like. For example, the thickness of the resistor 130 may be approximately 0.05 µm to 2 µm. In particular, when the thickness of the resistor 130 is 0.1 µm or greater, the crystallinity of the crystals constituting the resistor 130 (for example, the crystallinity of α-Cr) is improved. In addition, when the thickness of the resistor 130 is 1 µm or less, (i) cracks in the film and (ii) warpage of the film from the substrate 110, which are caused by the internal stress in the film constituting the resistor 130, are reduced.

Taking into consideration making lateral sensitivity less likely to occur and the countermeasures against wire breakage, the width of the resistor 130 is preferably 10 µm or greater and 100 µm or less. To be more specific, the width of the resistor 130 is preferably 10 µm or greater and 70 µm or less, and more preferably 10 µm or greater and 50 µm or less.

For example, when the resistor 130 is a Cr mixed phase film, the stability of the gauge characteristics can be improved by using alpha chromium (α-Cr), which is a stable crystal phase, as a main component. Moreover, for example, when the resistor 130 is a Cr mixed phase film, using α-Cr as the main component of the resistor 130 can bring the gauge factor of the strain gauge 100 to 10 or greater, and the temperature coefficient of gauge factor TCS and the temperature coefficient of resistance TCR to within a range of -1,000 ppm/°C to +1,000 ppm/°C. Here, the "main component" means that the component occupies 50% by weight or more of all materials constituting the resistor. From the viewpoint of improving the gauge characteristics, the resistor 130 preferably contains 80% by weight or more of α-Cr. Further, from the same viewpoint, the resistor 130 more preferably contains 90% by weight or more of α-Cr. Here, α-Cr is Cr having a bcc structure (body-centered cubic lattice structure).

When the resistor 130 is a Cr mixed phase film, CrN and Cr₂N contained in the Cr mixed phase film are preferably 20% by weight or less. When CrN and Cr₂N contained in the Cr mixed phase film are 20% by weight or less, a decrease in the gauge factor of the strain gauge 100 can be suppressed.

As the ratio between CrN and Cr₂N in the Cr mixed phase film, the proportion of Cr₂N is preferably 80% by weight or greater and less than 90% by weight relative to the total weight of CrN and Cr₂N. More preferably, as the ratio, the proportion of Cr₂N is more preferably 90% by weight or greater and less than 95% by weight relative to the total weight of CrN and Cr₂N. Cr₂N has semiconductor properties. Therefore, when the proportion of Cr₂N is 90% by weight or greater and less than 95% by weight, decrease in TCR (negative TCR) becomes more remarkable. Moreover, when the proportion of Cr₂N is 90% by weight or greater and less than 95% by weight, ceramization of the resistor 130 can be reduced. Therefore, it is possible to make brittle fracture of the resistor 130 less likely to occur.

Meanwhile, CrN has an advantage of being chemically stable. Including more CrN in the Cr mixed phase film can reduce the possibility of occurrence of unstable N. Therefore, a stable strain gauge can be obtained. Here, the term "unstable N " means trace N₂ or atomic N that may be present in the Cr mixed phase film. These types of unstable N may escape the film depending on the external environment (for example, a high-temperature environment). When unstable N escapes the film, the film stress in the Cr mixed phase film may change.

When the Cr mixed phase film is used as the material of the resistor 130 in the strain gauge 100, it is possible to achieve high sensitivity and size reduction. For example, while the output from existing strain gauges is approximately 0.04 mV/2 V, an output of 0.3 mV/2 V or greater can be obtained when the Cr mixed phase film is used as the material of the resistor 130. Further, while the size (gauge length × gauge width) of existing strain gauges is approximately 3 mm × 3 mm, the size (gauge length × gauge width) can be reduced to approximately 0.3 mm × 0.3 mm when the Cr mixed phase film is used as the material of the resistor 130.

The wiring 140 is provided on the substrate 110. The wiring 140 is electrically connected to the resistor 130 and the electrodes 150. The wiring 140 may be patterned in any shape, and is not limited to a straight line. The wiring 140 can have any width and any length. In FIG. 17, for convenience, the wiring 140 is shown with a stippled pattern that is less dense than that of the resistor 130.

The electrodes 150 are provided on the substrate 110. The electrodes 150 are electrically connected to the resistor 130 via the wiring 140. The electrodes 150 are formed in an approximately rectangular shape having a wider width than that of the wiring 140 in a plan view. The electrodes 150 are a pair of electrodes for outputting changes in the resistance value of the resistor 130 caused by a strain to the outside. For example, lead wires for external connection or the like are joined to the electrodes 150. A metal layer having a low resistance, such as copper and the like, or a metal layer having good solderability, such as gold and the like, may be laminated on the upper surface of the electrodes 150. Although the resistor 130, the wiring 140, and the electrodes 150 are designated by different reference numerals for convenience, they can be composed of the same material in the same process as an integrated body. In FIG. 17, for convenience, the electrodes 150 are shown with a stippled pattern having the same density as that of the wiring 140.

The cover layer 160 (protective layer) is provided as needed on the upper surface 110a of the substrate 110 to cover the resistor 130 and the wiring 140 and expose the electrodes 150. Examples of the material of the cover layer 160 include insulating resins, such as a PI resin, an epoxy resin, a PEEK resin, a PEN resin, a PET resin, a PPS resin, a composite resin (for example, silicone resin and polyolefin resin), and the like. The cover layer 160 may contain a filler and a pigment. The thickness of the cover layer 160 is not particularly limited and may be appropriately selected in accordance with the purpose. For example, the thickness of the cover layer 160 may be approximately 2 µm to 30 µm. By providing the cover layer 160, it is possible to inhibit mechanical damage to the resistor 130. Further, by providing the cover layer 160, it is possible to protect the resistor 130 from moisture and the like.

### [Method of Manufacturing Strain Gauge 100]

In the strain gauge 100 according to the present embodiment, the resistor 130, the wiring 140, the electrodes 150, and the cover layer 160 are formed on the substrate 110. Another layer (e.g., a functional layer described later) may be formed between the substrate 110 and the layers of these members.

Hereinafter, a method of manufacturing the strain gauge 100 will be described. In order to manufacture the strain gauge 100, first, the substrate 110 is prepared, and a metal layer (referred to as a metal layer A for convenience) is formed on the upper surface 110a of the substrate 110. The metal layer A is a layer that is ultimately patterned to become the resistor 130, the wiring 140, and the electrodes 150. Therefore, the material and thickness of the metal layer A are the same as the material and thickness of the resistor 130, the wiring 140, and the electrodes 150.

The metal layer A may be formed by, for example, a magnetron sputtering method using, as a target, a raw material from which the metal layer A can be formed. Instead of the magnetron sputtering method, the metal layer A may be formed by a reactive sputtering method, a vapor deposition method, an arc ion plating method, a pulsed laser deposition method, and the like. After the metal layer A is formed on the upper surface 110a of the substrate 110, the metal layer A is patterned into planar shapes that are the same as that of the resistor 130, the wiring 140, and the electrodes 150 of FIG. 17 by a well-known photolithography method.

The metal layer A may be formed after an underlying layer is formed on the upper surface 110a of the substrate 110. For example, a functional layer having a predetermined film thickness may be formed in vacuum on the upper surface 110a of the substrate 110 by a conventional sputtering method. By providing the underlying layer in this way, it is possible to stabilize the gauge characteristics of the strain gauge 100.

In the present application, the functional layer refers to a layer having a function of promoting the crystal growth of at least the upper metal layer A (resistor 130) to be deposited thereon. It is preferable that the functional layer further has a function of preventing oxidation of the metal layer A due to oxygen or moisture contained in the substrate 110 and/or a function of improving adhesiveness between the substrate 110 and the metal layer A. The functional layer may further have other functions.

The insulating resin film constituting the substrate 110 might contain oxygen and moisture, and Cr might form an autoxidized film. Therefore, especially when the metal layer A contains Cr, it is preferable to form a functional layer having a function of preventing oxidation of the metal layer A.

Thus, by providing the functional layer under the metal layer A in this way, it is possible to promote the crystal growth of the metal layer A, and to form the metal layer A composed of stable crystal phases. As a result, the stability of the gauge characteristics of the strain gauge 100 can be improved. In addition, diffusion of a material that is a constituent of the functional layer into the metal layer A improves the gauge characteristics of the strain gauge 100.

Examples of the material of the functional layer include one or more types of metals selected from the group consisting of Cr (chromium), Ti (titanium), V (vanadium), Nb (niobium), Ta (tantalum), Ni (nickel), Y (yttrium), Zr (zirconium), Hf (hafnium), Si (silicon), C (carbon), Zn (zinc), Cu (copper), Bi (bismuth), Fe (iron), Mo (molybdenum), W (tungsten), Ru (ruthenium), Rh (rhodium), Re (rhenium), Os (osmium), Ir (iridium), Pt (platinum), Pd (palladium), Ag (silver), Au (gold), Co (cobalt), Mn (manganese), and Al (aluminum), alloys of any of the metals in this group, or compounds of any of the metals in this group.

FIG. 19 is a cross-sectional view (part 2) illustrating the strain gauge according to the first embodiment. FIG. 19 shows a cross-sectional shape of the strain gauge 100 when the functional layer 120 is provided as an underlying layer of the resistor 130, the wiring 140, and the electrodes 150.

For example, the planar shape of the functional layer 120 may be patterned to be approximately the same as the planar shapes of the resistor 130, the wiring 140, and the electrodes 150. However, the planar shape of the functional layer 120, and that of the resistor 130, the wiring 140, and the electrodes 150 do not need to be approximately the same. For example, when forming the functional layer 120 from an insulating material, the functional layer 120 may be patterned to have a shape different from the planar shapes of the resistor 130, the wiring 140, and the electrodes 150. In this case, for example, the functional layer 120 may be formed solidly on the region where the resistor 130, the wiring 140, and the electrodes 150 are to be formed. Alternatively, the functional layer 120 may be formed solidly on the entirety of the upper surface of the substrate 110.

After forming the resistor 130, the wiring 140, and the electrodes 150, the cover layer 160 is formed on the upper surface 110a of the substrate 110 as needed. The cover layer 160 covers the resistor 130 and the wiring 140, but the electrodes 150 may be exposed from the cover layer 160. For example, the cover layer 160 can be formed by laminating a semi-cured thermosetting insulating resin film on the upper surface 110a of the substrate 110 to cover the resistor 130 and the wiring 140 and expose the electrodes 150, and then heating and curing the insulating resin film. The strain gauge 100 is completed through the above steps.

### <First Modification of First Embodiment>

In a first modification of the first embodiment, an example in which a biasing member is disposed between the pulse wave sensor and the cap part will be shown. In the first modification of the first embodiment, description of the same components as those of the embodiment already described may be omitted.

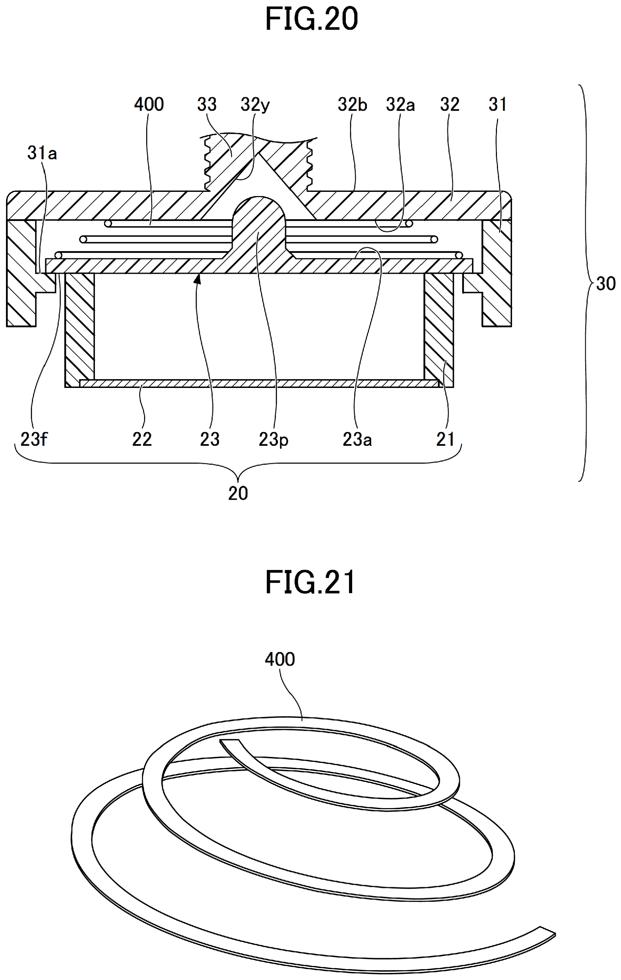

FIG. 20 is a view (part 1) for explaining the biasing member, and is a partial cross-sectional view showing the sensor part 10, the pulse wave sensor 20, and the cap part 32. FIG. 20 is different from the structure shown in FIG. 12 in that a biasing member 400 is disposed inside the sensor part 10 between a surface of the facing part 23 and a surface of the cap part 32 facing each other.

The biasing member 400 can be disposed between the first surface 23a of the facing part 23 and the first surface 32a of the cap part 32 to contact the first surface 23a of the facing part 23 and the first surface 32a of the cap part 32. By disposing the biasing member 400, it is possible to bias the pulse wave sensor 20 in a direction away from the first surface 32a of the cap part 32.

FIG. 21 is a view (part 2) for explaining the biasing member, and is an oblique view showing only the biasing member. As shown in FIG. 21, the biasing member 400 is, for example, a helical (spiral) leaf spring. The biasing member 400 can be disposed such that the pivot part 23p is approximately in the center of the biasing member 400 in a plan view. The biasing member 400 can be composed of, for example, metal, resin, rubber, and the like.

The biasing member 400 may be a conical spring or a cylindrical spring. When the biasing member is a conical spring, the height of the biasing member in the compressed state can be reduced from the height of the biasing member when it is a cylindrical spring. Therefore, the height of the sensor part 10 can be reduced. When the biasing member 400 is a conical spring, in order to stably dispose the biasing member 400, it is preferable to dispose it such that a part of the conical spring that has a small diameter is on the cap part 32 side.

As long as the pulse wave sensor 20 can be biased in a direction away from the first surface 32a of the cap part 32, the shape and material of the biasing member 400 do not matter.

By disposing the biasing member 400 in this way, it is possible to maintain the flange part 23f in contact with the stepped surface 31a even when vibration or the like is applied to the pulse wave sensor 20 in a state in which the pulse wave sensor is not in contact with a wrist or the like of a subject. Therefore, it is possible to prevent play between the pulse wave sensor 20 and the cap part 32. Thus, for example, it is possible to reduce the possibility that an abnormal sound occurs when the pulse wave measuring device 1 is carried, and the like.

### <Second Modification of First Embodiment>

In a second modification of the first embodiment, an example in which a cover member is attached to the first main surface side of the strain generating body of the pulse wave sensor will be shown. In the second modification of the first embodiment, description of the same components as those of the embodiments already described may be omitted.

A cover member may be provided to cover the entirety of the strain generating body 22 of the pulse wave sensor 20. For example, a separately prepared cover member composed of a material, such as silicone and the like, may be attached to the strain generating body 22 like a cap is attached, or a material, such as silicone and the like, may be integrally molded to be joined to the strain generating body 22 by such a method as insert molding and the like. Further, it is preferable that the cover member has a gap with respect to the strain generating body 22. A method of attaching the cover member to the pulse wave sensor 20 is not particularly limited.

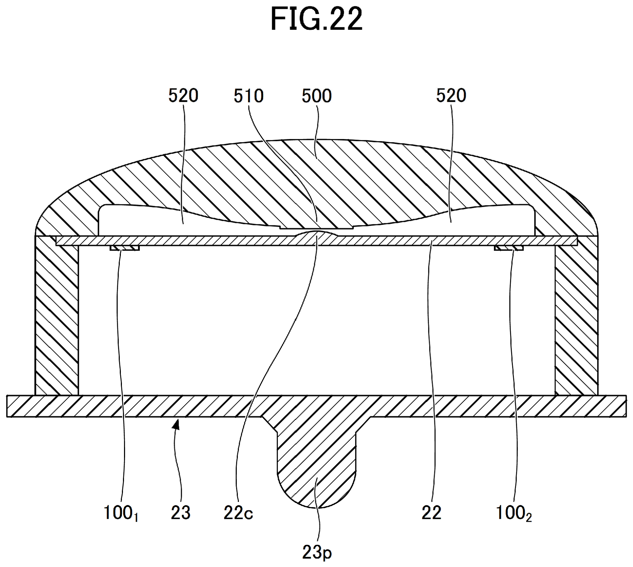

FIG. 22 is a cross-sectional view (part 1) showing a state in which the cover member is attached to the pulse wave sensor. In FIG. 22, as an example, a cover member 500 has a gap 520 with respect to the strain generating body 22, and a surface of the cover member 500 (on the side contacting the skin of a subject) has an approximately hemispherical shape. A projection 510 is provided in the center of the cover member 500, and the projection 510 and the load part 22c are provided to face each other.

The projection 510 and the strain generating body 22 (in the case of FIG. 22, the load part 22c) may be designed to be in contact with each other when no pressure is applied to the cover member 500, or may be designed to have a gap as shown in FIG. 22. That is, the projection 510 and the strain generating body 22 or the load part 22c may be separated from each other when no pressure is applied. Regardless of the presence or absence of the gap, the projection 510 is designed such that when the cover member 500 is pressed against the body to be measured, the projection 510 comes into contact with the strain generating body 22 (in the case of FIG. 22, the load part 22c) and transmits the pressure. With such a design, the pressure applied to the cover member 500 can be concentrated on the part of the strain generating body 22 that is in contact with the projection 510. Therefore, stress can be concentrated in a specific range of the strain generating body 22.

Further, by covering the strain generating body 22 with the cover member 500, it is possible to prevent litter, dust, and the like from entering the circular opening of the strain generating body 22. Further, even when the strain generating body 22 is composed of a metal, it is possible to prevent a subject from experiencing an allergic reaction to the metal.

The shape of the cover member 500 is not particularly limited. Yet, it is preferable to design the cover member 500 in a shape that allows pressure to be applied relatively uniformly to the load part 22c when the skin of the measurement target is pressed against the first main surface 22m of the strain generating body 22 at an angle other than the right angle. More specifically, it is preferable to design the cover member 500 such that pressure transmitted from the measurement target is applied relatively uniformly to the cover member 500 even when there is a change in the conditions of contact of the measurement target with the cover member 500 (that is, the magnitude of the pressing force that presses the cover member 500 against the measurement target, which part of the cover member 500 is in contact with the measurement target, and the angle at which the cover member 500 is pressed against the measurement target).

A preferable example of the above-described "shape that allows pressure to be applied relatively uniformly" is a hemispherical cover member 500 as shown in FIG. 22. As described above, the upper surface of the cover member 500 may have a curved surface that is highest above the center part of the first main surface 22m of the strain generating body 22 and becomes lower toward the periphery of the first main surface 22m of the strain generating body 22, when measured from the first main surface 22m of the strain generating body 22.

Other examples of the shape of the cover member 500 include, for example, a shape having a surface including a conic section of revolution, represented by (A) an ellipsoid (hemiellipsoid), (B) a super-ellipsoid (oval ellipsoid), (C) an ovoid, (D) a paraboloid of revolution shape, and the like. For example, the surface of the cover member 500 on the measurement target side may be such a surface including a conic section of revolution.

Note that the cover member 500 is not limited to the above-described (A) to (D), and may have a shape having a surface including a spline curve approximating a conic section of revolution (i.e., a spline surface), or a shape having a surface obtained by approximating a surface including a conic section of revolution with a straight line and a curve. To give a more broad definition, the cover member 500 may have a shape in which a line perpendicular to an arbitrary point on the surface of the cover member 500 on the measurement target side intersects a line perpendicular to the first main surface 22m of the strain generating body 22 and passing through the center of the first main surface 22m.

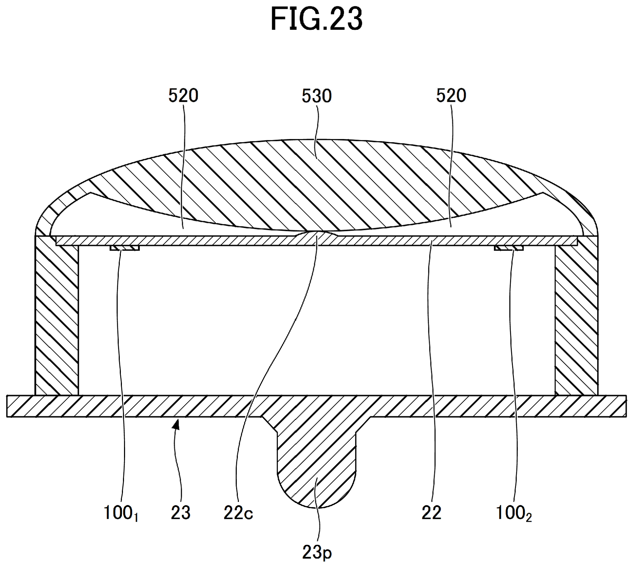

FIG. 23 is a cross-sectional view (part 2) showing a state in which the cover member is attached to the pulse wave sensor. A cover member 530 shown in FIG. 23 is different from the cover member 500 shown in FIG. 22 in that the cover member 530 has no such clearly projecting part as the projection 510 on a surface of the cover member 530 facing the strain generating body 22. The cover member 530 may be configured the same as the cover member 500 in any other points, or may be modified the same as the cover member 500.

At least a part of a surface of the cover member 530 facing the strain generating body 22 is composed of a curved surface curved toward the strain generating body 22. In this modification, the entirety of this curved surface of the cover member 530 serves as a projection. In the example of FIG. 23, the surface of the cover member 530 facing the strain generating body 22 is a curved surface of which the center part gently inclined toward the strain generating body 22. With such a design, the pressure applied to the cover member 530 can be concentrated on a part at which the cover member 530 and the strain generating body 22 contact each other (for example, the load part 22c of the strain generating body 22). Therefore, in the pulse wave sensor 20 having the cover member 530, stress can be concentrated in a specific range of the strain generating body 22. Further, like the cover member 500, use of the cover member 530 can also prevent liter, dust, and the like from entering from the circular opening of the strain generating body 22. Further, even when the strain generating body 22 is composed of metal, it is possible to prevent a subject from experiencing an allergic reaction to the metal.

Like the cover member 500, the shape of the cover member 530 is not limited to a hemispherical shape. For example, as described in the description of the cover member 500, the cover member 530 may have a shape having a surface including a conic section of revolution, a shape including a spline surface, or a shape having a surface obtained by approximating a surface including a conic section of revolution with a straight line and a curve.

Although preferred embodiments and the like have been described in detail above, the embodiments and the like described above are non-limiting, and various modifications and replacements are applicable to the embodiments and the like described above without departing from the scope described in the claims.

For example, in the above embodiment, the pivot part 23p is provided on the first surface 23a of the facing part 23, and the recessed portion 32y into which the pivot part 23p is insertable is opened in the first surface 32a of the cap part 32. However, the present invention is not limited to this, and the pivot part may be provided on the first surface 32a of the cap part 32, and the recessed portion into which the pivot part is insertable may be opened in the first surface 23a of the facing part 23. That is, the pivot part may be provided on one of a surface of the facing part 23 or a surface of the cap part 32, the surface of the facing part 23 and the surface of the cap part 32 facing each other, and the recessed portion into which the pivot part is insertable may be opened in the other.

The relationship between the internal screw part 61x of the first disk part 61 and the external screw part 33 of the movable part 30 may be reversed. That is, the external screw part may be provided on the first disk part 61 and the internal screw part may be provided on the movable part 30, and both may be threadedly engaged. When the external screw part 33 is provided on the movable part 30 side, it is preferable that the external screw part 33 is composed of a left-hand screw. Thus, when the rotating part 60 is rotated clockwise, the movable part 30 moves downward, and when the rotating part 60 is rotated counterclockwise, the movable part 30 moves upward. This is a configuration easy for a user of the pulse wave measuring device 1 to understand and operate. On the other hand, when the external screw part is provided on the first disk part 61 side, it is preferable that the external screw part is composed of a right-handed screw. As a result, these parts move the same as described above, providing a configuration easy for a user of the pulse wave measuring device 1 to understand and operate.

The structure of the pulse wave sensor 20 is not limited to that shown in FIG. 14 and the like, and may be any structure. For example, a structure in which no slit is provided around a beam part may be used.

The invention is defined by the claims as follows.

### REFERENCE SIGNS LIST

1: pulse wave measuring device, 10: sensor part, 20: pulse wave sensor, 21: housing, 21x: groove, 22: strain generating body, 22m: first main surface, 22n: second main surface, 23: facing part, 23a: first surface, 23f: flange part, 23p: pivot part, 23x: through-hole, 23y: notch part, 30: movable part, 31: sensor holding part, 31a: stepped surface, 31b: positioning part, 31x, 31y: groove, 32: cap part, 32x: through-hole, 33: external screw part, 40: fixed part, 41: cylindrical part, 41a: stepped surface, 41b: inner surface, 41c: projection, 41x: through-hole, 41y: notch part, 42: flange part, 42x: through-hole, 50: connecting part, 50f: flange part, 50x: through-hole, 60: rotating part, 61: first disk part, 61x: internal screw part, 61y: through-hole, 62: second disk part, 62a: stepped surface, 62x: through-hole, 62y: projection, 80: attaching part, 81: first curved member, 82: second curved member, 82z: cut-out part, 83: cap part, 84, 85: biasing member, 86: swing shaft, 87: first operation part, 88: second operation part, 90: wiring board, 100₁, 100₂, 100₃, 100₄: strain gauge, 300: radial artery, 310: skin, 400: biasing member, 500, 530: cover member, 510: projection, 520 gap

## Claims

1. A pulse wave measuring device, comprising:
a sensor part including a pulse wave sensor; and
an attaching part connected outside the sensor part and attachable to a subject,
wherein the attaching part includes a first curved member and a second curved member that are curved in opposite directions and face each other to be attachable to a wrist of the subject, and can shift between a closed state and an opened state,
the sensor part is disposed at one end of the first curved member in a longitudinal direction of the first curved member,
the second curved member includes a region of which a width in a transverse direction of the second curved member is narrower than a width of the first curved member in a transverse direction of the first curved member, and
the second curved member has an asymmetrical shape with respect to an imaginary line that bisects a maximum width part of the second curved member in the transverse direction and extends in a longitudinal direction of the second curved member.

2. The pulse wave measuring device according to claim 1,
wherein the second curved member includes a cut-out part obtained by cutting the second curved member from one end of the second curved member in the transverse direction toward the imaginary line, and
the region is adjacent to the cut-out part in the transverse direction of the second curved member.

3. The pulse wave measuring device according to claim 2,
wherein the cut-out part is located on a side toward the sensor part in the longitudinal direction of the second curved member.

4. The pulse wave measuring device according to claim 3,
wherein in a bottom view, a center of the sensor part is located closer to the cut-out part than the imaginary line is in the transverse direction of the second curved member.

5. The pulse wave measuring device according to any one of claims 1 to 4,
wherein a width of the second curved member in the transverse direction on a side of the second curved member that is farther from the sensor part than the region is in the longitudinal direction of the second curved member is same as the width of the first curved member in the transverse direction.

6. The pulse wave measuring device according to any one of claims 1 to 5,
wherein the sensor part includes:
a fixed part including a cylindrical part;
a movable part accommodated inside the cylindrical part; and
a rotating part closing one opening of the cylindrical part and configured to rotate with respect to the fixed part about a center axis of the cylindrical part serving as a rotation axis,
wherein the movable part includes:
a sensor holding part;
the pulse wave sensor; and
a cap part fixed to an other end side of the sensor holding part in an axial direction of the sensor holding part and connected to the rotating part,
wherein the pulse wave sensor includes:
a housing;
a strain generating body provided on one side of the housing; and
a facing part provided on the other side of the housing,
the pulse wave sensor being held inside the sensor holding part such that the strain generating body is exposed from one end side of the sensor holding part in the axial direction of the sensor holding part,
wherein a pivot part is provided on one of a surface of the facing part or a surface of the cap part, the surface of the facing part and the surface of the cap part facing each other, and a recessed portion into which the pivot part is insertable is provided in the other of the surface of the facing part or the surface of the cap part,
wherein when the pivot part and the recessed portion come into contact with each other, the pulse wave sensor becomes swingable on a fulcrum, which is a contact part at which the pivot part and the recessed portion contact each other,
wherein the strain generating body is exposed from the other opening of the cylindrical part, and
wherein the movable part reciprocates in an axial direction of the cylindrical part along with rotation of the rotating part, without rotating with respect to the fixed part.

7. The pulse wave measuring device according to claim 6,
wherein the movable part includes a first screw part projecting from the cap part in a direction opposite to the strain generating body,
the rotating part includes a first disk part including a second screw part projecting toward the strain generating body,
one of the first screw part or the second screw part is an external screw part, and the other is an internal screw part,
the external screw part and the internal screw part are threadedly engaged with each other in a rotatable manner, and
when the first disk part rotates, a threadedly engaged state of the external screw part and the internal screw part changes, and the movable part reciprocates in the axial direction of the cylindrical part.

8. The pulse wave measuring device according to claim 7,
wherein the rotating part further includes a second disk part having a diameter smaller than that of the first disk part,
the fixed part includes a cylindrical connecting part having both ends that are open,
the connecting part includes, at an end of the connecting part on a side where the first disk part is, a first flange part projecting radially inward from an outer surface of the connecting part,
the second disk part is accommodated in the connecting part and fixed to the first disk part with the first flange part sandwiched between the second disk part and the first disk part, and
the connecting part is fixed to the cylindrical part.

9. The pulse wave measuring device according to any one of claims 6 to 8,
wherein the facing part includes a second flange part projecting to a radially outer side of the housing from an outer surface of the housing,
the sensor holding part includes a stepped surface projecting toward a center axis of the sensor holding part,
the second flange part is disposed between the stepped surface and the cap part, and
the second flange part serves as a stopper configured to prevent the pulse wave sensor from slipping out from the sensor holding part.

10. The pulse wave measuring device according to claim 9,
wherein the sensor holding part includes a positioning part provided on an upper side of the stepped surface and projecting from an inner surface of the sensor holding part toward the center axis of the sensor holding part,
the facing part includes a notch part recessed from an outer periphery of the facing part toward a center of the facing part, and
the pulse wave sensor is held inside the sensor holding part with the notch part aligned with the positioning part, and is prevented from rotating relative to the sensor holding part.

11. The pulse wave measuring device according to any one of claims 6 to 10,
wherein the sensor holding part includes a groove recessed from an outer surface of the sensor holding part toward a center of the sensor holding part,
the groove has an elongated shape where a longitudinal direction of the groove is aligned with the axial direction of the sensor holding part,
the cylindrical part includes a projection projecting from an inner surface of the cylindrical part toward the center axis of the cylindrical part, and
the projection and the groove are fitted with each other such that the sensor holding part is prevented from rotating relative to the cylindrical part, and a moving range of the sensor holding part is restricted to a range defined by a length of the groove in the longitudinal direction.

12. The pulse wave measuring device according to any one of claims 6 to 11,
wherein a biasing member configured to bias the pulse wave sensor in a direction away from the cap part is disposed between the surface of the facing part and the surface of the cap part facing each other.

13. The pulse wave measuring device according to any one of claims 6 to 12,
wherein the strain generating body includes a plurality of strain gauges, and
the pulse wave sensor detects a pulse wave based on changes in resistance values of resistors of the plurality of strain gauges.

14. The pulse wave measuring device according to claim 13,
wherein the plurality of strain gauges include a pair of strain gauges configured to detect a compressive strain of the strain generating body and another pair of strain gauges configured to detect a tensile strain of the strain generating body,
the pair of strain gauges and the another pair of strain gauges are connected to form respective sides of one bridge circuit, and
a signal indicative of the pulse wave is generated by the bridge circuit.

15. The pulse wave measuring device according to claim 13 or 14,
wherein the strain generating body includes a first main surface and a second main surface located on a side opposite to the first main surface,
the plurality of strain gauges are disposed on the second main surface, and
a cover member is attached to the first main surface.

16. The pulse wave measuring device according to any one of claims 1 to 15,
wherein the first curved member includes a wiring board disposed between the one end and the other end of the first curved member in the longitudinal direction, and
a component contributing to detection of a pulse wave is disposed on the wiring board.

## Patentansprüche

1. Pulswellenmessvorrichtung, umfassend:
einen Sensorteil, der einen Pulswellensensor beinhaltet; und
einen Befestigungsteil, der außerhalb des Sensorteils verbunden und an einem Subjekt befestigbar ist,
wobei der Befestigungsteil ein erstes gekrümmtes Element und ein zweites gekrümmtes Element beinhaltet, die in entgegengesetzte Richtungen gekrümmt sind und einander gegenüberliegen, um an einem Handgelenk des Subjekts befestigbar zu sein, und zwischen einem geschlossenen und einem offenen Zustand wechseln können,
der Sensorteil an einem Ende des ersten gekrümmten Elements in einer Längsrichtung des ersten gekrümmten Elements angeordnet ist,
das zweite gekrümmte Element eine Region beinhaltet, von der eine Breite in einer Querrichtung des zweiten gekrümmten Elements schmaler ist als eine Breite des ersten gekrümmten Elements in einer Querrichtung des ersten gekrümmten Elements, und
das zweite gekrümmte Element eine asymmetrische Form in Bezug auf eine imaginäre Linie aufweist, die einen Teil von maximaler Breite des zweiten gekrümmten Elements in der Querrichtung halbiert und sich in eine Längsrichtung des zweiten gekrümmten Elements erstreckt.

2. Pulswellenmessvorrichtung nach Anspruch 1,
wobei das zweite gekrümmte Element einen Ausschnittteil beinhaltet, der durch Schneiden des zweiten gekrümmten Elements von einem Ende des zweiten gekrümmten Elements in die Querrichtung zu der imaginären Linie hin erhalten wird, und
die Region angrenzend an den Ausschnittteil in der Querrichtung des zweiten gekrümmten Elements liegt.

3. Pulswellenmessvorrichtung nach Anspruch 2,
wobei der Ausschnittteil sich auf einer Seite zu dem Sensorteil hin in der Längsrichtung des zweiten gekrümmten Elements befindet.

4. Pulswellenmessvorrichtung nach Anspruch 3,
wobei in einer Ansicht von unten, sich eine Mitte des Sensorteils näher an dem Ausschnittteil befindet als es die imaginäre Linie in der Querrichtung des zweiten gekrümmten Elements ist.

5. Pulswellenmessvorrichtung nach einem der Ansprüche 1 bis 4,
wobei eine Breite des zweiten gekrümmten Elements in der Querrichtung auf einer Seite des zweiten gekrümmten Elements, das weiter entfernt von dem Sensorteil ist als es die Region in der Längsrichtung des zweiten gekrümmten Elements ist, dieselbe ist wie die Breite des ersten gekrümmten Elements in der Querrichtung.

6. Pulswellenmessvorrichtung nach einem der Ansprüche 1 bis 5,
wobei der Sensorteil Folgendes beinhaltet:
einen fixierten Teil, der einen zylindrischen Teil beinhaltet;
einen beweglichen Teil, der im Inneren des zylindrischen Teils aufgenommen ist; und
einen sich drehenden Teil, der eine Öffnung des zylindrischen Teils schließt und dazu konfiguriert ist, sich in Bezug auf den fixierten Teil um eine Mittelachse des zylindrischen Teils zu drehen, die als Drehachse dient,
wobei der bewegliche Teil Folgendes beinhaltet:
einen Sensorhalteteil;
den Pulswellensensor; und
einen Deckelteil, der an einer anderen Endseite des Sensorhalteteils in einer axialen Richtung des Sensorhalteteils fixiert ist und mit dem sich drehenden Teil verbunden ist;
wobei der Pulswellensensor Folgendes beinhaltet:
ein Gehäuse;
einen Spannung-erzeugenden Körper, der auf einer Seite des Gehäuses bereitgestellt ist; und
einen gegenüberliegenden Teil, der auf der anderen Seite des Gehäuses bereitgestellt ist,
wobei der Pulswellensensor im Inneren des Sensorhalteteils derart gehalten wird, dass der Spannung-erzeugende Körper aus einer Endseite des Sensorhalteteils in die axiale Richtung des Sensorhalteteils freiliegt,
wobei ein Schwenkteil auf einer von einer Oberfläche des gegenüberliegenden Teils oder einer Oberfläche des Deckelteils bereitgestellt ist, wobei die Oberfläche des gegenüberliegenden Teils und die Oberfläche des Deckelteils einander gegenüberliegen, und ein vertiefter Abschnitt, in den der Schwenkteil einsetzbar ist, in der anderen von der Oberfläche des gegenüberliegenden Teils und der Oberfläche des Deckelteils bereitgestellt ist,
wobei, wenn der Schwenkteil und der vertiefte Abschnitt in Kontakt miteinander kommen, der Pulswellensensor auf einem Angelpunkt schwenkbar wird, der ein Kontaktteil ist, an dem der Schwenkteil und der vertiefte Abschnitt einander kontaktieren,
wobei der Spannung-erzeugende Körper aus der anderen Öffnung des zylindrischen Teils freiliegt, und
wobei der bewegliche Teil sich in einer axialen Richtung des zylindrischen Teils zusammen mit einer Drehung des sich drehenden Teils hin- und herbewegt, ohne sich in Bezug auf den fixierten Teil zu drehen.

7. Pulswellenmessvorrichtung nach Anspruch 6,
wobei der bewegliche Teil einen ersten Schraubenteil beinhaltet, der von dem Deckelteil in eine Richtung entgegengesetzt zu dem Spannung-erzeugenden Körper vorspringt,
der sich drehende Teil einen ersten Scheibenteil beinhaltet, der einen zweiten Schraubenteil beinhaltet, der zu dem Spannung-erzeugenden Körper hin vorspringt,
einer von dem ersten Schraubenteil oder dem zweiten Schraubenteil ein äußerer Schraubenteil ist und der andere ein innerer Schraubenteil ist,
der äußere Schraubenteil und der innere Schraubenteil auf drehbare Weise gewindemäßig miteinander in Eingriff sind, und
wenn sich der erste Scheibenteil dreht, sich ein gewindemäßiger Eingriffszustand des äußeren Schraubenteils und des inneren Schraubenteils ändert und sich der bewegliche Teil in der axialen Richtung des zylindrischen Teils hin- und herbewegt.

8. Pulswellenmessvorrichtung nach Anspruch 7,
wobei der sich drehende Teil ferner einen zweiten Scheibenteil beinhaltet, der einen kleineren Durchmesser als der erste Scheibenteil aufweist,
der fixierte Teil einen zylindrischen Verbindungsteil beinhaltet, dessen beide Enden offen sind,
der Verbindungsteil an einem Ende des Verbindungsteils auf einer Seite, wo der erste Scheibenteil ist, einen ersten Flanschteil beinhaltet, der von einer Außenoberfläche des Verbindungsteils radial nach innen vorspringt,
der zweite Scheibenteil in dem Verbindungsteil aufgenommen und an dem ersten Scheibenteil mit dem ersten Flanschteil fixiert ist, das zwischen dem zweiten Scheibenteil und dem ersten Scheibenteil sandwichartig angeordnet ist.
der Verbindungsteil an dem zylindrischen Teil fixiert ist.

9. Pulswellenmessvorrichtung nach einem der Ansprüche 6 bis 8,
wobei der gegenüberliegende Teil einen zweiten Flanschteil beinhaltet, der von einer Außenoberfläche des Gehäuses zu einer radialen Außenseite des Gehäuses vorspringt,
der Sensorhalteteil eine gestufte Oberfläche beinhaltet, die zu einer Mittelachse des Sensorhalteteils hin vorspringt,
der zweite Flanschteil zwischen der gestuften Oberfläche und dem Deckelteil angeordnet ist, und
der zweite Flanschteil als ein Anschlag dient, der dazu konfiguriert ist, zu verhindern, dass der Pulswellensensor aus dem Sensorhalteteil herausrutscht.

10. Pulswellenmessvorrichtung nach Anspruch 9,
wobei der Sensorhalteteil einen Positionierungsteil beinhaltet, der auf einer oberen Seite der gestuften Oberfläche bereitgestellt ist und von einer Innenoberfläche des Sensorhalteteils zu der Mittelachse des Sensorhalteteils hin vorspringt,
der gegenüberliegende Teil einen Nutteil beinhaltet, der von einem Außenumfang des gegenüberliegenden Teils zu einer Mitte des gegenüberliegenden Teils hin vertieft ist, und
der Pulswellensensor im Inneren des Sensorhalteteils gehalten wird, indem der Nutteil mit dem Positionierungsteil ausgerichtet ist, und verhindert wird, dass er sich relativ zu dem Sensorhalteteil dreht.

11. Pulswellenmessvorrichtung nach einem der Ansprüche 6 bis 10,
wobei der Sensorhalteteil eine Rille beinhaltet, die von einer Außenoberfläche des Sensorhalteteils zu einer Mitte des Sensorhalteteils hin vertieft ist,
die Rille eine längliche Form aufweist, wo eine Längsrichtung der Rille mit der axialen Richtung des Sensorhalteteils ausgerichtet ist,
der zylindrische Teil einen Vorsprung beinhaltet, der von einer Innenoberfläche des zylindrischen Teils zu der Mittelachse des zylindrischen Teils hin vorspringt, und
der Vorsprung und die Rille derart ineinander eingepasst sind, dass verhindert wird, dass der Sensorhalteteil sich relativ zu dem zylindrischen Teil dreht, und ein Bewegungsbereich des Sensorhalteteils auf einen Bereich begrenzt wird, der durch eine Länge der Rille in der Längsrichtung definiert ist.

12. Pulswellenmessvorrichtung nach einem der Ansprüche 6 bis 11,
wobei ein Vorspannelement, das dazu konfiguriert ist, den Pulswellensensor in eine Richtung weg von dem Deckelteil vorzuspannen, zwischen der Oberfläche des gegenüberliegenden Teils und der Oberfläche des Deckelteils, die einander gegenüberliegen, angeordnet ist.

13. Pulswellenmessvorrichtung nach einem der Ansprüche 6 bis 12,
wobei der Spannung-erzeugende Körper eine Vielzahl von Spannungsmessern beinhaltet, und
der Pulswellensensor eine Pulswelle basierend auf Änderungen von Widerstandswerten von Widerständen der Vielzahl von Spannungsmessern erkennt.

14. Pulswellenmessvorrichtung nach Anspruch 13,
wobei die Vielzahl von Spannungsmessern ein Paar von Spannungsmessern, die dazu konfiguriert sind, eine Druckspannung des Spannung-erzeugenden Körpers zu erkennen, und ein anderes Paar von Spannungsmessern, die dazu konfiguriert sind, eine Zugspannung des Spannung-erzeugenden Körpers zu erkennen, beinhaltet,
das Paar von Spannungsmessern und das andere Paar von Spannungsmessern dazu verbunden sind, jeweilige Seiten einer Brückenschaltung auszubilden, und
ein Signal, das angebend für die Pulswelle ist, durch die Brückenschaltung erzeugt wird.

15. Pulswellenmessvorrichtung nach Anspruch 13 oder 14,
wobei der Spannung-erzeugende Körper eine erste Hauptoberfläche und eine zweite Hauptoberfläche, die sich auf einer Seite gegenüber der ersten Hauptoberfläche befindet, beinhaltet,
die Vielzahl von Spannungsmessern auf der zweiten Hauptoberfläche angeordnet sind, und
ein Abdeckelement an der ersten Hauptoberfläche befestigt ist.

16. Pulswellenmessvorrichtung nach einem der Ansprüche 1 bis 15,
wobei das erste gekrümmte Element eine Leiterplatte beinhaltet, die zwischen dem einen Ende und dem anderen Ende des ersten gekrümmten Elements in der Längsrichtung angeordnet ist, und
eine Komponente, die zur Erkennung einer Pulswelle beiträgt, auf der Leiterplatte angeordnet ist.

## Revendications

1. Dispositif de mesure d'onde pulsatile, comprenant :
une partie capteur incluant un capteur d'onde pulsatile ; et
une partie fixation reliée à l'extérieur de la partie capteur et pouvant être fixée à un sujet,
dans lequel la partie fixation inclut un premier élément incurvé et un deuxième élément incurvé qui sont incurvés dans des directions opposées et se font face de façon à pouvoir être fixés à un poignet du sujet, et peuvent passer d'un état fermé à un état ouvert,
la partie capteur est disposée à une extrémité du premier élément incurvé dans une direction longitudinale du premier élément incurvé,
le deuxième élément incurvé inclut une région dont une largeur dans une direction transversale du deuxième élément incurvé est plus étroite qu'une largeur du premier élément incurvé dans une direction transversale du premier élément incurvé, et
le deuxième élément incurvé a une forme asymétrique par rapport à une ligne imaginaire qui divise en deux une partie de largeur maximale du deuxième élément incurvé dans la direction transversale et s'étend dans une direction longitudinale du deuxième élément incurvé.

2. Dispositif de mesure d'onde pulsatile selon la revendication 1,
dans lequel le deuxième élément incurvé inclut une partie découpée obtenue en découpant le deuxième élément incurvé à partir d'une extrémité du deuxième élément incurvé dans la direction transversale vers la ligne imaginaire, et
la région est adjacente à la partie découpée dans la direction transversale du deuxième élément incurvé.

3. Dispositif de mesure d'onde pulsatile selon la revendication 2,
dans lequel la partie découpée est située sur un côté vers la partie capteur dans la direction longitudinale du deuxième élément incurvé.

4. Dispositif de mesure d'onde pulsatile selon la revendication 3,
dans lequel dans une vue de dessous, un centre de la partie capteur est situé plus près de la partie découpée que ne l'est la ligne imaginaire dans la direction transversale du deuxième élément incurvé.

5. Dispositif de mesure d'onde pulsatile selon l'une quelconque des revendications 1 à 4,
dans lequel une largeur du deuxième élément incurvé dans la direction transversale sur un côté du deuxième élément incurvé qui est plus éloigné de la partie capteur que ne l'est la région dans la direction longitudinale du deuxième élément incurvé est identique à la largeur du premier élément incurvé dans la direction transversale.

6. Dispositif de mesure d'onde pulsatile selon l'une quelconque des revendications 1 à 5,
dans lequel la partie capteur inclut :
une partie fixe incluant une partie cylindrique ;
une partie mobile logée à l'intérieur de la partie cylindrique ; et
une partie rotative fermant une ouverture de la partie cylindrique et configurée pour tourner par rapport à la partie fixe autour d'un axe central de la partie cylindrique servant d'axe de rotation,
dans lequel la partie mobile inclut :
une partie supportant le capteur ;
le capteur d'onde pulsatile ; et
une partie capuchon fixée à un autre côté d'extrémité de la partie supportant le capteur dans une direction axiale de la partie supportant le capteur et reliée à la partie rotative,
dans lequel le capteur d'onde pulsatile inclut :
un boîtier ;
un corps générateur de contrainte prévu sur un côté du boîtier ; et
une partie frontale prévue sur l'autre côté du boîtier,
le capteur d'onde pulsatile étant maintenu à l'intérieur de la partie supportant le capteur de telle sorte que le corps générateur de contrainte est exposé depuis un côté d'extrémité de la partie supportant le capteur dans la direction axiale de la partie supportant le capteur,
dans lequel une partie pivot est prévue sur une d'une surface de la partie frontale ou d'une surface de la partie capuchon, la surface de la partie frontale et la surface de la partie capuchon se faisant face, et une portion en retrait dans laquelle la partie pivot peut être insérée est prévue dans l'autre de la surface de la partie frontale ou de la surface de la partie capuchon,
dans lequel lorsque la partie pivot et la portion en retrait viennent en contact l'une avec l'autre, le capteur d'onde pulsatile devient oscillant sur un point d'appui, qui est une partie de contact au niveau de laquelle la partie pivot et la portion en retrait sont en contact l'une avec l'autre,
dans lequel le corps générateur de contrainte est exposé à partir de l'autre ouverture de la partie cylindrique, et
dans lequel la partie mobile effectue un mouvement de va-et-vient dans une direction axiale de la partie cylindrique parallèlement à la rotation de la partie rotative, sans tourner par rapport à la partie fixe.

7. Dispositif de mesure d'onde pulsatile selon la revendication 6,
dans lequel la partie mobile inclut une première partie de vis faisant saillie à partir de la partie capuchon dans une direction opposée au corps générateur de contrainte,
la partie rotative inclut une première partie de disque incluant une deuxième partie de vis faisant saillie vers le corps générateur de contrainte,
l'une de la première partie de vis ou de la deuxième partie de vis est une partie de vis externe, et l'autre est une partie de vis interne,
la partie de vis externe et la partie de vis interne sont en prise par filetage l'une avec l'autre d'une manière rotative, et
lorsque la première partie de disque tourne, un état de mise en prise par filetage de la partie de vis externe et la partie de vis interne change, et la partie mobile effectue un mouvement de va-et-vient dans la direction axiale de la partie cylindrique.

8. Dispositif de mesure d'onde pulsatile selon la revendication 7,
dans lequel la partie rotative inclut en outre une deuxième partie de disque présentant un diamètre inférieur à celui de la première partie de disque,
la partie fixe inclut une partie de raccordement cylindrique dont les deux extrémités sont ouvertes,
la partie de raccordement inclut, au niveau d'une extrémité de la partie de raccordement sur un côté où se trouve la première partie de disque, une première partie de bride faisant saillie radialement vers l'intérieur à partir d'une surface externe de la partie de raccordement,
la deuxième partie de disque est logée dans la partie de raccordement et fixée à la première partie de disque avec la première partie de bride prise en sandwich entre la deuxième partie de disque et la première partie de disque, et
la partie de raccordement est fixée à la partie cylindrique.

9. Dispositif de mesure d'onde pulsatile selon l'une quelconque des revendications 6 à 8,
dans lequel la partie frontale inclut une deuxième partie de bride faisant saillie vers un côté radialement externe du boîtier à partir d'une surface externe du boîtier,
la partie supportant le capteur inclut une surface étagée faisant saillie vers un axe central de la partie supportant le capteur,
la deuxième partie de bride est disposée entre la surface étagée et la partie capuchon, et
la deuxième partie de bride sert de butoir configuré pour empêcher le capteur d'onde pulsatile de glisser hors de la partie supportant le capteur.

10. Dispositif de mesure d'onde pulsatile selon la revendication 9,
dans lequel la partie supportant le capteur inclut une partie de positionnement prévue sur un côté supérieur de la surface étagée et faisant saillie à partir d'une surface interne de la partie supportant le capteur vers l'axe central de la partie supportant le capteur,
la partie frontale inclut une partie encoche en retrait par rapport à une périphérie externe de la partie frontale vers un centre de la partie frontale, et
le capteur d'onde pulsatile est maintenu à l'intérieur de la partie supportant le capteur avec la partie encoche alignée avec la partie de positionnement, et est dans l'impossibilité de tourner par rapport à la partie supportant le capteur.

11. Dispositif de mesure d'onde pulsatile selon l'une quelconque des revendications 6 à 10,
dans lequel la partie supportant le capteur inclut une rainure en retrait par rapport à une surface externe de la partie supportant le capteur vers un centre de la partie supportant le capteur,
la rainure présente une forme allongée où une direction longitudinale de la rainure est alignée avec la direction axiale de la partie supportant le capteur,
la partie cylindrique inclut une saillie faisant saillie à partir d'une surface interne de la partie cylindrique vers l'axe central de la partie cylindrique, et
la saillie et la rainure sont assemblées l'une à l'autre de sorte que la partie supportant le capteur ne peut pas tourner par rapport à la partie cylindrique, et une plage de mouvement de la partie supportant le capteur est limitée à une plage définie par une longueur de la rainure dans la direction longitudinale.

12. Dispositif de mesure d'onde pulsatile selon l'une quelconque des revendications 6 à 11,
dans lequel un élément de sollicitation configuré pour solliciter le capteur d'onde pulsatile dans une direction s'écartant de la partie capuchon est disposé entre la surface de la partie frontale et la surface de la partie capuchon se faisant face.

13. Dispositif de mesure d'onde pulsatile selon l'une quelconque des revendications 6 à 12,
dans lequel le corps générateur de contrainte inclut une pluralité de jauges de contrainte, et
le capteur d'onde pulsatile détecte une onde pulsatile sur la base de changements de valeurs de résistance de résistances de la pluralité de jauges de contrainte.

14. Dispositif de mesure d'onde pulsatile selon la revendication 13,
dans lequel la pluralité de jauges de contrainte inclut une paire de jauges de contrainte configurée pour détecter une contrainte de compression du corps générateur de contrainte et une autre paire de jauges de contrainte configurée pour détecter une contrainte de traction du corps générateur de contrainte,
la paire de jauges de contrainte et l'autre paire de jauges de contrainte sont reliées afin de former des côtés respectifs d'un circuit en pont, et
un signal indiquant que l'onde pulsatile est générée par le circuit en pont.

15. Dispositif de mesure d'onde pulsatile selon la revendication 13 ou 14, dans lequel le corps générateur de contrainte inclut une première surface principale et une deuxième surface principale située sur un côté opposé à la première surface principale,
la pluralité de jauges de contrainte est disposée sur la deuxième surface principale, et
un élément de recouvrement est fixé à la première surface principale.

16. Dispositif de mesure d'onde pulsatile selon l'une quelconque des revendications 1 à 15,
dans lequel le premier élément incurvé inclut une carte de câblage disposée entre l'une des extrémités et l'autre extrémité du premier élément incurvé dans la direction longitudinale, et
un composant contribuant à la détection d'une onde pulsatile est disposé sur la carte de câblage.
